# EUROPEAN PATENT APPLICATION

(11) **EP 4 538 294 A1**
(43) Date of publication of application: **16.04.2025**
(21) Application number: 23819054.0
(22) Date of filing: 05.06.2023
(51) Int. Cl.: C07K 16/46, C07K 16/30, C07K 16/28, C07K 19/00, A61K 39/395, A61P 35/00, C12N 15/13, C12N 15/63, C12N 5/10, C12P 21/00

(54) **MULTI-SPECIFIC ANTIBODY TARGETING BCMA, GPRC5D AND T CELLS AND APPLICATION THEREOF**

(30) Priority: 06.06.2022 CN 202210631397
(71) Applicant: Shandong Simcere Biopharmaceutical Co., Ltd., Shandong 264006 (CN)
(72) Inventor: FU, Yayuan, Shanghai 201318 (CN); YOU, Shumei, Shanghai 201318 (CN); LIAO, Jingli, Shanghai 201318 (CN); CAO, Zhuoxiao, Shanghai 201318 (CN); TANG, Renhong, Shanghai 201318 (CN)
(74) Representative: Mewburn Ellis LLP
(86) International application number: PCT/CN2023/098241
(87) International publication number: WO 2023/236889

(57) **Abstract**

The present application relates to a multi-specific binding molecule targeting BCMA, GPRC5D and a T cell receptor. In particular, the present application discloses a multi-specific antibody against BCMA, GPRC5D and CD3, which can bind to a tumor surface antigen while activating T cells, thereby promoting the specific killing of tumor cells, in particular BCMA-positive or GPRC5D-positive multiple myeloma, by T cells. The present application further provides a preparation method for and the application of the multi-specific binding molecule.

## Description

### Cross Reference to Related Application

The present application claims the right of priority for Chinese patent application No. 202210631397.9, filed on 06 June 2022, which is incorporated herein by reference in its entirety for all purposes.

### Technical Field

The present disclosure relates to the field of biomedicine, in particular to a multi-specific antibody targeting BCMA, GPRC5D and T cells and the application thereof.

### Background

Multiple myeloma (MM) is a plasma cell malignant tumor characterized by the unrestricted proliferation of plasma cells in the bone marrow like tumor cells, accompanied by secretion of monoclonal immunoglobulins, which causes a series of clinical manifestations such as multiple osteolytic damage, hypercalcemia, anemia, kidney damage and repeated infections. Multiple myeloma accounts for 1% of all tumors and 10%-15% of hematologic malignancies. The ratio of male to female is 1.6 : 1, and most patients are over 40 years old. The treatment of multiple myeloma includes chemotherapy, hematopoietic stem cell transplantation, etc. Among them, immunomodulators represented by lenalidomide and protease inhibitors represented by bortezomib, used either alone or in combination, have shown good efficacy, and have become conventional treatment methods for patients with multiple myeloma. However, multiple myeloma is still considered as an incurable disease. At present, treatment methods can only relieve the symptoms of multiple myeloma, but none of them can completely eliminate the tumor. Almost all patients will eventually relapse. Therefore, there is an urgent need for new treatment options.

In recent years, breakthroughs have been made in the use of CAR-T therapy or targeted ADC therapy for multiple myeloma. In particular, CAR T with B cell maturation antigen (BCMA) as a target and antibody-drug conjugates (ADCs) targeting BCMA have shown positive clinical results in the treatment of multiple myeloma, and a variety of drugs have been approved for marketing.

B cell maturation antigen (BCMA), also known as CD269 or TNFRSF17, is a member of the tumor necrosis factor receptor superfamily and was first identified in the early 1990s. BCMA is mainly expressed on the surface of mature B lymphocytes and plasma cells, and is a landmark protein for the maturation of B lymphocytes. Structurally, BCMA consists of three main domains: an extracellular segment (aa1-54), a transmembrane region (aa55-77) and an intracellular segment (aa78-184). B cell activating factor (BAFF) and proliferation inducing ligand (APRIL) are the main ligands of BCMA, which transmit cell stimulation signals by interacting with BCMA, activate TRAP-dependent NF-κB and JNK pathways and increase the proliferation and survival rate of B cells. BCMA is mainly expressed on the surface of mature B lymphocytes and plasma cells, but hardly expressed in other tissue cells. Moreover, BCMA is highly expressed in tumor cells of most patients with multiple myeloma, so BCMA is an ideal therapeutic target for multiple myeloma.

However, for patients with BCMA-negative multiple myeloma or multiple myeloma with low expression of BCMA and patients with relapse after BCMA targeted therapy, there is an urgent need for more and more effective treatment methods. Therefore, for the treatment of multiple myeloma, we still need to find other new targets with more potentials.

### Summary of the Invention

In order to solve the above-mentioned technical problem, the present application provides the following technical solutions:
In a first aspect, the present disclosure provides a multi-specific binding molecule, comprising:
(a) a first antigen binding molecule (ABM1) that specifically binds to a first tumor-associated antigen (TAA1);
(b) a second antigen binding molecule (ABM2) that specifically binds to a second tumor-associated antigen (TAA2); and
(c) a third antigen binding molecule (ABM3) that specifically binds to an antigen expressed on a human immune cell.

In a second aspect, the present disclosure provides an isolated nucleic acid, encoding the aforementioned multi-specific binding molecule.

In a third aspect, the present disclosure provides a recombinant vector, comprising the aforementioned isolated nucleic acid.

In a fourth aspect, the present disclosure provides a host cell, comprising the aforementioned isolated nucleic acid or the aforementioned recombinant vector.

In a fifth aspect, the present disclosure provides a method for preparing a multi-specific binding molecule, comprising: culturing the aforementioned host cell under appropriate conditions, and isolating the multi-specific binding molecule.

In a sixth aspect, the present disclosure provides a pharmaceutical composition, comprising the aforementioned multi-specific binding molecule, the aforementioned isolated nucleic acid, the aforementioned recombinant vector, the aforementioned host cell, or a product prepared according to the aforementioned method, and one or more pharmaceutically acceptable carriers, diluents, buffers or excipients.

In a seventh aspect, the present disclosure provides a method for activating T cells, comprising: administering to a subject a therapeutically effective amount of the aforementioned multi-specific binding molecule or pharmaceutical composition.

In an eighth aspect, the present disclosure provides the aforementioned multi-specific binding molecule, the aforementioned isolated nucleic acid, the aforementioned host cell, a product prepared by the aforementioned method or the aforementioned pharmaceutical composition, for use in the preparation of a drug for preventing and/or treating cancers, or for use in the treatment and/or prevention of cancers.

In a ninth aspect, the present disclosure provides a method for treating and/or preventing cancers, comprising: administering to a subject a therapeutically effective amount of the aforementioned multi-specific binding molecule, the aforementioned isolated nucleic acid, the aforementioned host cell, a product prepared by the aforementioned method or the aforementioned pharmaceutical composition.

Beneficial effects: the development of dual-targeted multi-specific antibodies that target GPRC5D and BCMA and related biological products has the advantages of covering more populations with tumors, possibly bringing more thorough tumor clearance and delaying tumor recurrence, and has great potential clinical needs.

### Brief Description of the Figures

FIG. 1 shows the schematic structure diagrams of trispecific antibody molecules and positive and negative control molecules.
FIG. 2A shows the FACS detection result of a CHOK1 stable transformation cell line expressing human GPRC5D protein.
FIG. 2B shows the FACS detection result of a CHOK1 stable transformation cell line expressing monkey GPRC5D protein.
FIG. 3 shows the FACS result of the BCMA expression quantity of Flp-inCHO-human BCMA cells.
FIG. 4A shows the detection of the binding activity of trispecific antibodies against human BCMA-his protein by ELISA.
FIG. 4B shows the detection of the binding activity of trispecific antibodies against monkey BCMA-his protein by ELISA.
FIG. 5A shows the detection of the binding activity of trispecific antibodies against CHOK1-human GPRC5D cells by Cell-based ELISA.
FIG. 5B shows the detection of the binding activity of trispecific antibodies against CHOK1-monkey GPRC5D cells by Cell-based ELISA.
FIG. 6A shows the detection of the binding activity of trispecific antibodies against human CD3e/d-his protein by ELISA.
FIG. 6B shows the detection of the binding activity of trispecific antibodies against human CD3e-his protein by ELISA.
FIG. 7 shows the detection of the binding activity of trispecific antibodies against Flp-inCHO-human BCMA cells by FACS.
FIG. 8 shows the detection of the binding activity of trispecific antibodies against CHOK1-human GPRC5D cells by FACS.
FIG. 9 shows the detection of the binding activity of trispecific antibodies against H929 cells by FACS.
FIG. 10 shows the detection of the binding activity of trispecific antibodies against Jurkat cells by FACS.
FIG. 11A shows the detection of the binding activity of trispecific antibodies against human PBMC by FACS.
FIG. 11B shows the detection of the binding activity of trispecific antibodies against monkey PBMC by FACS.
FIG. 12A shows the activation activity of trispecific antibodies against Jurkat-NFAT-NanoLuc cells under the mediation of H929 cells.
FIG. 12B shows the activation activity of trispecific antibodies against Jurkat-NFAT-NanoLuc cells under the mediation of K562 cells.
FIG. 13 shows the killing effect of T cells on H929-Luc cells under the mediation of trispecific antibodies.
FIG. 14A shows the release of cytokine TNFα when T cells kill H929-Luc under the mediation of trispecific antibodies.
FIG. 14B shows the release of cytokine IFNγ when T cells kill H929-Luc under the mediation of trispecific antibodies.
FIG. 15 shows the killing effect of T cells on RPMI8226-Luc cells under the mediation of trispecific antibodies.
FIG. 16A shows the release of cytokine TNFα when T cells kill RPMI8226-Luc under the mediation of trispecific antibodies.
FIG. 16B shows the release of cytokine IFNγ when T cells kill RPMI8226-Luc under the mediation of trispecific antibodies.
FIG. 17 shows the killing effect of T cells on Molp-8-Luc or Molp-8 cells under the mediation of trispecific antibodies.
FIG. 18A shows the release of cytokine TNFα when T cells kill Molp-8-Luc or Molp-8 under the mediation of trispecific antibodies.
FIG. 18B shows the release of cytokine IFNγ when T cells kill Molp-8-Luc or Molp-8 under the mediation of trispecific antibodies.

### Definition and Description of Terminology

Unless defined otherwise in the present disclosure, scientific and technical terms related to the present disclosure shall have the meanings understood by one of ordinary skill in the art.

In addition, unless otherwise specified herein, terms in the singular form herein shall include that in the plural form, and terms in the plural form shall include that in the singular form. More specifically, as used in the description and the appended claims, the singular forms "a/an" and "this" include plural referents, unless otherwise clearly stated.

The terms "include", "comprise" and "have" are used interchangeably herein and are intended to indicate the inclusiveness of the solution, meaning that the solution can have other elements than those listed. Furthermore, it should be understood that the description of "include", "comprise" and "have" as used herein also provides the solution of "consist of".

The term "and/or" as used herein includes the meanings of "and", "or" and "all or any other combination of elements linked by the term".

The term "BCMA" herein is the abbreviated name of B cell maturation antigen, which is a member of the tumor necrosis factor receptor family. BCMA is mainly expressed on the surface of late B cells, short-lived proliferative plasma cells and long-lived plasma cells, and is not expressed in naive B cells, CD34-positive hematopoietic stem cells and other normal tissue cells, but is highly expressed in MM cells. BCMA plays a key role in the survival, proliferation, metastasis and drug resistance of MM cells by mediating downstream signaling pathways, thereby being an ideal target antigen for the treatment of MM. Exemplary human BCMA sequences can be seen in GenBank Protein Accession No: NP_001183.2.

The term "GPRC5D" herein refers to G protein coupled receptor family C group 5 member D, which is an orphan receptor and is a seven-transmembrane protein. GPRC5D is highly expressed on the surface of primary multiple myeloma cells, while its expression in normal tissues is limited to the hair follicle area. Studies have shown that 65% of patients with multiple myeloma have a GPRC5D expression threshold exceeding 50%. With this characteristic, GPRC5D has become a potential target for the treatment of MM. Exemplary human GPRC5D sequences can be seen in GenBank Protein Accession No: NP_061124.1.

The term "CD3" herein refers to an antigen that is a portion of a multimolecular T cell receptor (TCR) on a T cell, and consists of a homodimer or heterodimer formed by two of the following four receptor chains: CD3ε, CD3δ, CD3ζ and CD3γ. Human CD3-ε (hCD3ε) comprises UniProtKB/Swiss-Prot: the amino acid sequence in P07766.2. Human CD3-δ (hCD3δ) comprises UniProtKB/Swiss-Prot: the amino acid sequence in P04234.1. Therefore, the "CD3" refers to human CD3, unless clearly stated that it is from a non-human species, such as "mouse CD3" and "monkey CD3".

The term "tumor-associated antigen" or "TAA" herein refers to a molecule (typically a protein, a carbohydrate, a lipid or some combinations thereof) expressed completely or as a fragment (such as MHC/peptide) on the surface of cancerous B cells, and may be used for preferentially targeting a pharmacological agent to cancerous B cells. As used herein, the term "cancerous B cells" refers to B cells that are undergoing or have undergone uncontrolled proliferation. In some examples, TAA is a marker expressed by both normal cells and cancer cells, such as a lineage marker, such as CD19 on B cells. In some instances, TAA is a B cell surface molecule that is overexpressed in cancerous B cells compared to normal B cells, such as overexpressed by 1 time, 2 times, 3 times or more times compared to normal B cells, for example, BCMA and GPRC5D that have a much higher expression on cancerous B cells than on normal tissues. In some examples, TAA is a cell surface molecule that is improperly synthesized in cancerous B cells, for example, a molecule containing a deletion, addition or mutation compared to a molecule expressed on normal B cells. In some examples, TAA is only expressed completely or as a fragment (such as MHC/peptide) on the cell surface of cancerous B cells, and not synthesized or expressed on the surface of normal cells. Therefore, the term "TAA" covers B cell antigens specific for cancer cells, sometimes referred to as "tumor specific antigen" (TSA) in the art.

The term "specifically bind" herein means that an antigen binding molecule (e.g., an antibody) generally specifically binds to an antigen and substantially the same antigen with a high affinity, but does not bind to an unrelated antigen with a high affinity. Affinity is generally reflected by the equilibrium dissociation constant (KD), where lower KD indicates higher affinity. Taking an antibody as an example, a high affinity generally means having a KD of about 10⁻⁶ M or less, 10⁻⁷ M or less, about 10⁻⁸ M or less or about 10⁻⁹ M or less. KD is calculated as follows: KD = Kd/Ka, where Kd represents the dissociation rate and Ka represents the binding rate. The equilibrium dissociation constant KD can be measured using well-known methods in the art, such as surface plasmon resonance (e.g., Biacore) or equilibrium dialysis assay.

The term "antigen binding molecule" is used herein in the broadest sense and refers to a molecule that specifically binds to an antigen. Exemplarily, the antigen binding molecule includes, but is not limited to, an antibody or an antibody mimetic. The "antibody mimetic" refers to an organic compound or a binding domain that can specifically bind to an antigen, but is not structurally related to an antibody. Exemplarily, the antibody mimetic includes, but is not limited to, affibody, affitin, affilin, a designed ankyrin repeat protein (DARPin), a nucleic acid aptamer or a Kunitz-type domain peptide.

The term "antibody" is used herein in the broadest sense and refers to a polypeptide or a combination of polypeptides comprising sufficient sequences from a heavy chain variable region of an immunoglobulin and/or sufficient sequences from a light chain variable region of an immunoglobulin to be able to specifically bind to an antigen. The "antibody" herein covers various forms and various structures as long as they exhibit the desired antigen binding activity. The "antibody" herein includes an alternative protein scaffold or artificial scaffold with grafted complementarity determining regions (CDRs) or CDR derivatives. Such scaffolds include antibody-derived scaffolds comprising mutations introduced, e.g., to stabilize the three-dimensional structure of the antibody, and fully synthetic scaffolds comprising, e.g., biocompatible polymers. See, for example, Korndorfer, I. P., Beste, G. & Skerra, A. (2003). Proteins, 53, 121-129.; Roque, A. C. A., Lowe, C. R. & Taipa, M. A. Antibodies and genetically engineered related molecules: production and purification. Biotechnol. Prog. 20, 639-654 (2004). Such scaffolds may also include non-antibody-derived scaffolds, for example, scaffold proteins known in the art which can be used for grafting CDRs, including but not limited to tenascin, fibronectin, a peptide aptamer, *etc.*

The term "antibody" includes an intact antibody and any antigen binding fragment (i.e., "antigen binding moiety") or single chain thereof. The "antibody" refers to a glycoprotein comprising at least two heavy (H) chains and two light (L) chains interconnected by a disulfide bond, or an antigen binding moiety thereof. Each heavy chain consists of a heavy chain variable region (abbreviated herein as VH) and a heavy chain constant region. The heavy chain constant region consists of three domains, CH1, CH2 and CH3. Each light chain consists of a light chain variable region (abbreviated herein as VL) and a light chain constant region. The light chain constant region consists of one domain, CL. The VH and VL regions can be further subdivided into hypervariable regions known as complementarity determining regions (CDRs), which are interspersed among more conserved regions known as framework regions (FRs). Each VH and VL consists of three CDRs and four FRs, which are arranged in the following order from amino terminus to carboxyl terminus: FR1, CDR1, FR2, CDR2, FR3, CDR3, FR4. The variable regions of the heavy and light chains contain a binding domain that can interact with an antigen. The constant region of an antibody can mediate the binding of an immunoglobulin to a host tissue or factor, and the host tissue or factor includes various cells of the immune system (e.g., effector cells) and a first component of the classical complement system (C1q). Since the amino acid composition and arrangement sequence of the heavy chain constant region of an immunoglobulin are different, the antigenicity of the immunoglobulin is also different. Accordingly, "immunoglobulin" herein can be divided into five classes, or isotypes of immunoglobulins, namely IgM, IgD, IgG, IgA and IgE, and the corresponding heavy chains thereof are a µ chain, a δ chain, a γ chain, an α chain and an ε chain, respectively. The same class of Ig can also be divided into different subclasses according to the differences in the amino acid composition of the hinge region thereof and the number and position of heavy chain disulfide bonds. For example, IgG can be divided into IgG1, IgG2, IgG3 and IgG4, and IgA can be divided into IgA1 and IgA2. The light chains are divided into a κ chain or a λ chain by the difference in the constant region. Each Ig class of the five Ig classes can have either a κ chain or a λ chain.

The "antibody" herein also includes an antibody that does not comprise light chains, for example, heavy-chain antibodies (HCAbs) produced from camelids, such as Camelus dromedarius, Camelus bactrianus, Lama glama, Lama guanicoe and Vicugna pacos, and Ig new antigen receptors (IgNARs) found in Chondrichthyes such as sharks.

The term "antibody" herein can be derived from any animals, including but not limited to humans and non-human animals. The non-human animals can be selected from primates, mammals, rodents and vertebrates, such as camelids, Lama glama, Lama guanicoe, Vicugna pacos, goats, rabbits, mice, rats or Chondrichthyes (such as sharks).

The term "heavy chain antibody" herein refers to an antibody that lacks light chains of a conventional antibody. The term specifically includes, but is not limited to, a homodimeric antibody comprising a VH antigen binding domain and CH2 and CH3 constant domains in the absence of a CH1 domain.

The term "nanobody" herein refers to a natural heavy chain antibody without light chains in camel, etc., and cloning the variable region thereof can obtain a single domain antibody, also known as VHH (Variable domain of heavy chain of heavy chain antibody), which only consists of a heavy chain variable region, and is the smallest functional antigen binding fragment.

The terms "VHH domain", "nanobody" and "single domain antibody" (sdAb) herein have the same meaning and are used interchangeably, and refer to a single domain antibody consisting of only one heavy chain variable region constructed by cloning the variable region of a heavy chain antibody, which is the smallest antigen binding fragment with full functionality. Generally, a single domain antibody consisting of only one heavy chain variable region is constructed by obtaining a heavy chain antibody that naturally lacks light chains and heavy chain constant region 1 (CH1), and then cloning the heavy chain variable region of the antibody.

Further descriptions of the "heavy chain antibody", "single domain antibody", "VHH domain" and "nanobody" can be seen in: Hamers-Casterman et al., Nature. 363, 446-8(1993). Naturally occurring antibodies devoid of light chains.; the review article by Muyldermans (J Biotechnol. 2001 Jun; 74(4):277-302. Single domain camel antibodies: current status); and the following patent applications mentioned as general background art: WO 94/04678, WO 95/04079 and WO 96/34103; WO 94/25591, WO 99/37681, WO 00/40968, WO 00/43507, WO 00/65057, WO 01/40310, WO 01/44301, EP 1134231 and WO 02/48193; WO 97/49805, WO 01/21817, WO 03/035694, WO 03/054016 and WO 03/055527; WO 03/050531; WO 01/90190; WO 03/025020; and WO 04/041867, WO 04/041862, WO 04/041865, WO 04/041863, WO 04/062551, WO 05/044858, WO 06/40153, WO 06/079372, WO 06/122786, WO 06/122787 and WO 06/122825, and other prior art mentioned in these applications.

The term "multi-specific" herein refers to the ability of an antibody or an antigen binding fragment thereof to bind to, for example, different antigens or at least two different epitopes on the same antigen. Therefore, the terms such as "bispecific", "trispecific" and "tetraspecific" refer to the number of different epitopes to which an antibody can bind. For example, a conventional monospecific IgG-type antibody has two identical antigen binding sites (paratopes) and thus can only bind to the same epitope (rather than bind to different epitopes). In contrast, a multi-specific antibody has at least two different types of paratopes/binding sites and thus can bind to at least two different epitopes. As described herein, "complementarity determining region" refers to an antigen binding site of an antibody. Furthermore, single "specific" may refer to one, two, three or more identical complementarity determining regions in a single antibody (the actual number of complementarity determining regions/binding sites in a single antibody molecule is referred to as "valent"). For example, a single natural IgG antibody is monospecific and bivalent because it has two identical paratopes. Accordingly, a multi-specific antibody comprises at least two (different) complementarity determining regions/binding sites. Therefore, the term "multi-specific antibody" refers to an antibody that has more than one paratope and has the ability to bind to two or more different epitopes. The term "multi-specific antibody" particularly includes the bispecific antibody as defined above, and generally also includes a protein, e.g., an antibody or a scaffold specifically binding to three or more different epitopes, i.e., an antibody having three or more paratopes/binding sites.

The term "valent" herein refers to the presence of a defined number of antigen binding domains in an antibody/antigen binding molecule. Therefore, the terms "trivalent", "tetravalent", "pentavalent" and "hexavalent" refer to the presence of three antigen binding domains, four antigen binding domains, five antigen binding domains and six antigen binding domains in an antibody/an antigen binding molecule, respectively.

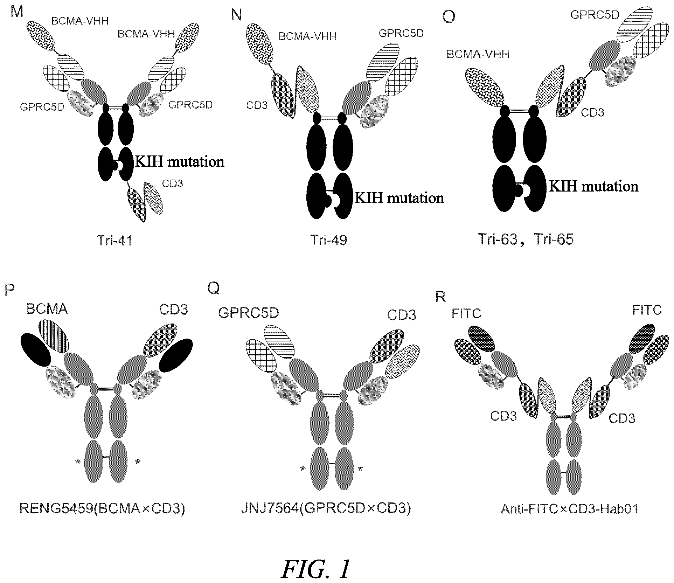

The term "trivalent" used herein refers to an antigen binding molecule having three antigen binding domains. The multi-specific antigen binding molecule of the present disclosure is trispecific and specifically binds to TAA1, TAA2 and CD3. Therefore, the trivalent multi-specific binding molecule of the present disclosure has at least three antigen binding domains that each bind to a different antigen. Examples of the trivalent multi-specific binding molecule of the present disclosure are schematically shown in FIG. 1N or 1O.

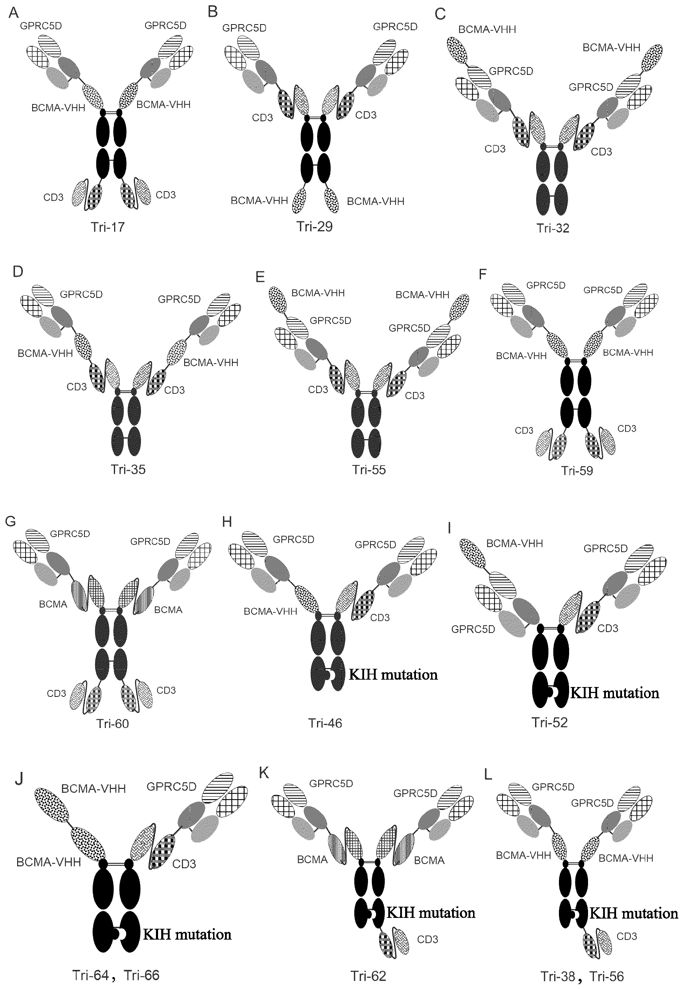

The term "tetravalent" used herein refers to an antigen binding molecule having four antigen binding domains. The multi-specific antigen binding molecule of the present disclosure is trispecific and specifically binds to TAA1, TAA2 and CD3. Therefore, the tetravalent multi-specific binding molecule of the present disclosure generally has two antigen binding domains that bind to the same antigen (e.g., TAA1 or TAA2) and two antigen binding domains that each bind to a separate antigen (e.g., CD3, and TAA1 or TAA2). Examples of the tetravalent multi-specific binding molecule of the present disclosure are schematically shown in FIGs. 1H-1J.

The term "pentavalent" used herein refers to an antigen binding molecule having five antigen binding domains. The multi-specific binding molecule of the present disclosure is trispecific and specifically binds to TAA1, TAA2 and CD3. Therefore, the pentavalent multi-specific binding molecule of the present disclosure generally has two pairs of antigen binding domains that each bind to the same antigen, and a single antigen binding domain that binds to a third antigen (e.g., CD3). Examples of the pentavalent multi-specific binding molecule of the present disclosure are schematically shown in FIGs. 1K-1M.

The term "hexavalent" used herein refers to an antigen binding molecule having six antigen binding domains. The multi-specific binding molecule of the present disclosure is trispecific and specifically binds to TAA1, TAA2 and CD3. Although different configurations (e.g., three antigen binding domains that bind to TAA1, two antigen binding domains that bind to TAA2, and one antigen binding domain that binds to CD3, or three antigen binding domains that bind to TAA1, two antigen binding domains that bind to CD3, and one antigen binding domain that binds to TAA2) fall within the scope of the present disclosure, the hexavalent multi-specific binding molecule of the present disclosure generally has three pairs of two antigen binding domains that each bind to the same antigen. Examples of the hexavalent multi-specific binding molecule of the present disclosure are schematically shown in FIGs. 1A-1G.

The terms "full-length antibody", "complete antibody" and "intact antibody" herein are used interchangeably herein and mean that they have a structure substantially similar to that of a natural antibody.

The terms "antigen binding fragment" and "antibody fragment" herein are used interchangeably herein, which do not possess the full structure of an intact antibody, and only comprise a part of an intact antibody or a variant of the part, wherein the part of the intact antibody or the variant of the part has the ability to bind to an antigen. Exemplarily, the "antigen binding fragment" or "antibody fragment" herein includes, but is not limited to, Fab, F(ab')2, Fab', Fab'-SH, Fd, Fv, scFv, diabody and a single domain antibody.

The term "linker" herein refers to linking polypeptide sequences for linking a protein domain, and generally has a certain degree of flexibility. The use of linker does not result in loss of the original function of the protein domain.

The term "chimeric antibody" herein refers to an antibody that has a variable sequence of an immunoglobulin from a source organism (such as a rat, a mouse, a rabbit or a Vicugna pacos) and a constant region of an immunoglobulin from a different organism (such as human). Methods for producing the chimeric antibody are known in the art. See, for example, Morrison, 1985, Science 229(4719): 1202-1207. Transfectomas Provide Novel Chimeric Antibodies; and Gillies et al., J Immunol Methods. 1989 Dec 20; 125(1-2): 191-202; the above documents are incorporated herein by reference. See also patent US 5807715 A.

The term "humanized antibody" herein refers to a non-human antibody that has been genetically engineered, with amino acid sequences modified to improve the homology with the sequences of a human antibody. Generally speaking, all or part of the CDR regions of a humanized antibody come from a non-human antibody (a donor antibody), and all or part of the non-CDR regions (for example, a variable region FR and/or a constant region) come from a human immunoglobulin (a receptor antibody). A humanized antibody usually retains or partially retains the expected properties of a donor antibody, including but not limited to, antigen specificity, affinity, reactivity, ability to enhance immune cell activity or ability to enhance immune response, etc.

The term "fully human antibody" herein refers to an antibody having variable regions in which both the FRs and CDRs are derived from human germline immunoglobulin sequences. Furthermore, if the antibody comprises a constant region, the constant region is also derived from human germline immunoglobulin sequences. The "fully human antibody" herein may include amino acid residues not encoded by human germline immunoglobulin sequences (for example, mutations introduced by random or site-specific mutagenesis in vitro or by somatic mutation in vivo). However, the "fully human antibody" herein does not include an antibody in which CDR sequences derived from the germline of another mammalian species (e.g., a mouse) have been grafted onto human framework sequences.

The term "variable region" herein refers to a region in the heavy or light chain of an antibody that is involved in enabling the antibody to bind to an antigen. The "heavy chain variable region", "VH" and "HCVR" are used interchangeably, and the "light chain variable region", "VL" and "LCVR" are used interchangeably. The variable regions of the heavy and light chains of a natural antibody generally have similar structures, and each region comprises four conserved framework regions (FRs) and three hypervariable regions (HVRs). See, for example, Kindt et al., Kuby Immunology, 6th edition, W.H.Freeman and Co., p. 91 (2007). A single VH or VL may be sufficient to confer antigen binding specificity.

The terms "complementarity determining region" and "CDR" herein are used interchangeably and generally refer to hypervariable regions (HVRs) found in both light and heavy chain variable domains. The more conserved portions in variable domains are called framework regions (FRs). As understood in the art, the amino acid positions representing the hypervariable regions of an antibody can vary according to the context and various definitions known in the art. Some positions within variable domains can be considered heterozygous hypervariable positions because these positions can be considered to be within the hypervariable regions under one set of criteria (such as IMGT or KABAT) but outside the hypervariable regions under a different set of criteria (such as KABAT or IMGT). One or more of these positions may also be found in extended hypervariable regions. The present disclosure includes an antibody comprising modifications in these heterozygous hypervariable positions. The heavy chain variable region CDR can be abbreviated as HCDR, and the light chain variable region CDR can be abbreviated as LCDR. The variable domains of natural heavy and light chains respectively comprise four framework regions predominantly in a sheet configuration, which are linked by three CDRs (CDR1, CDR2 and CDR3) that form a loop linking the sheet structure, and in some cases form part of the sheet structure. The CDRs in each chain are closely held together in the order of FR1-CDR1-FR2-CDR2-FR3-CDR3-FR4 by the FR region, and together with the CDRs from other antibody chains, contribute to the formation of an antigen binding site of an antibody.

The "CDRs" herein can be marked and defined by well-known methods in the art, including but not limited to Kabat numbering system, Chothia numbering system or IMGT numbering system. The tool websites used include, but are not limited to, AbRSA website (http://cao.labshare.cn/AbRSA/cdrs.php), abYsis website (www.abysis.org/abysis/sequence_input/key_annotation/key_annotation.cgi) and IMGT website (http://www.imgt.org/3Dstructure-DB/cgi/DomainGapAlign.cgi#results). The CDRs herein include overlaps and subsets of amino acid residues defined in different ways.

The term "Kabat numbering system" herein generally refers to the immunoglobulin alignment and numbering system proposed by Elvin A. Kabat (see, for example, Kabat et al., Sequences of Proteins of Immunological Interest, 5th Ed. Public Health Service, National Institutes of Health, Bethesda, Md., 1991).

The term "Chothia numbering system" herein generally refers to the immunoglobulin numbering system proposed by Chothia et al., which is a classical rule for identifying the boundaries of CDR regions based on the location of structural loop regions (see, for example, Chothia, C., & Lesk, A. M. (1987). Canonical structures for the hypervariable regions of immunoglobulins. Journal of molecular biology, 196(4), 901-917.).

The term "IMGT numbering system" herein generally refers to the numbering system based on the international ImMunoGeneTics information system (IMGT) initiated by Lefranc et al., see Lefranc, M. P., Pommié, C., Ruiz, M., Giudicelli, V., Foulquier, E., Truong, L., ... & Lefranc, G. (2003). IMGT unique numbering for immunoglobulin and T cell receptor variable domains and Ig superfamily V-like domains. Developmental & Comparative Immunology, 27(1), 55-77.

The term "heavy chain constant region" herein refers to the carboxyl-terminal portion of the heavy chain of an antibody, which is not directly involved in the binding of the antibody to an antigen, but exhibits effector functions, such as interaction with Fc receptors, and has a more conserved amino acid sequence relative to the variable domain of the antibody. The "heavy chain constant region" can be selected from a CH1 domain, a hinge region, a CH2 domain, a CH3 domain, or a variant or fragment thereof. The "heavy chain constant region" includes "full-length heavy chain constant region" and "heavy chain constant region fragment", the former has a structure substantially similar to that of a natural antibody constant region, while the latter only includes "a portion of the full-length heavy chain constant region". Exemplarily, a typical "full-length antibody heavy chain constant region" consists of CH1 domain-hinge region-CH2 domain-CH3 domain; when the antibody is IgE, it also includes a CH4 domain; and when the antibody is a heavy chain antibody, it does not include the CH1 domain. Exemplarily, a typical "heavy chain constant region fragment" can be selected from Fc or a CH3 domain.

The term "light chain constant region" herein refers to the carboxyl-terminal portion of the light chain of an antibody, which is not directly involved in the binding of the antibody to an antigen. The light chain constant region can be selected from a constant κ domain or a constant λ domain.

The term "Fc region" herein is used to define the C-terminal region of an antibody heavy chain that contains at least a portion of the constant region. The term includes a native sequence Fc region and a variant Fc region. Exemplarily, human IgG heavy chain Fc region can extend from Cys226 or Pro230 to the carboxyl terminus of the heavy chain. However, an antibody generated from a host cell may undergo post-translational cleavage whereby one or more, particularly one or two amino acids are cleaved off from the C-terminus of the heavy chain. Therefore, by expression of a specific nucleic acid molecule encoding a full-length heavy chain, the antibody generated from a host cell can include the full-length heavy chain, or a cleavage variant of the full-length heavy chain. This may be the case when the last two C-terminal amino acids of the heavy chain are glycine (G446) and lysine (K447, numbering according to the Kabat EU index). Therefore, the C-terminal lysine (Lys447), or the C-terminal glycine (Gly446) and lysine (Lys447) of the Fc region may or may not be present. Typically, an IgG Fc region comprises IgG CH2 and IgG CH3 domains, and optionally, on this basis, the IgG Fc region can also comprise a complete or partial hinge region, but does not comprise a CH1 domain. The "CH2 domain" of a human IgG Fc region generally extends from an amino acid residue at about position 231 to an amino acid residue at about position 340. In one embodiment, a carbohydrate chain is attached to the CH2 domain. The CH2 domain herein can be a native sequence CH2 domain or a variant CH2 domain. The "CH3 domain" comprises the stretch of residues at the C-terminus of the CH2 domain in the Fc region (i.e., from an amino acid residue at about position 341 to an amino acid residue at about position 447 of IgG). The CH3 region herein can be a native sequence CH3 domain or a variant CH3 domain (e.g., a CH3 domain having a "knob" introduced in one chain thereof and a "hole" correspondingly introduced in the other chain thereof; see US Patent No. 5,821,333, which is expressly incorporated herein by reference). As described herein, such variant CH3 domains can be used to facilitate heterodimerization of two non-identical antibody heavy chains.

Unless otherwise specified herein, the numbering of amino acid residues in an Fc region or a constant region is according to the EU numbering system, also known as the EU index, as described in Kabat et al., Sequences of Proteins of Immunological Interest, 5th edition. Public Health Service, National Institutes of Health, Bethesda, MD, 1991.

The term "Fc variant" herein refers to changes in the structure or function of Fc caused by one or more amino acid substitution, insertion or deletion mutations at appropriate positions on the Fc. The "interaction between Fc variants" refers to the space-filling effect, electrostatic steering, hydrogen bonding effect, hydrophobic effect, etc. formed between Fc variants that have been subjected to a mutation design. The interaction between Fc variants contributes to the formation of a stable heterodimeric protein. A preferred mutation design is a mutation design in a "Knob-into-hole (KIH)" form.

The mutation design technique of Fc variants has been widely used in the field to prepare bispecific antibodies or heterodimeric Fc fusion protein forms. Representative examples include a "Knob-into-hole" form proposed by Cater et al. (Ridgway, J. B., Presta, L. G., & Carter, P. (1996). 'Knobs-into-holes' engineering of antibody CH3 domains for heavy chain heterodimerization. Protein Engineering, Design and Selection, 9(7), 617-621.); an Fc-containing heterodimer form formed by using Electrostatic Steering by the technician in Amgen (US 20100286374 A1); a heterodimer form formed by IgG/IgA strand exchange (SEEDbodies) proposed by Jonathan H.Davis et al. (Davis, J. H., Aperlo, C., Li, Y., Kurosawa, E., Lan, Y., Lo, K. M., & Huston, J. S. (2010). SEEDbodies: fusion proteins based on strand-exchange engineered domain (SEED) CH3 heterodimers in an Fc analog platform for asymmetric binders or immunofusions and bispecific antibodies. Protein Engineering, Design & Selection, 23(4), 195-202.); a bispecific molecule formed by the platform technique DuoBody of Genmab (Gramer, M. J., van den Bremer, E. T., van Kampen, M. D., Kundu, A., Kopfmann, P., Etter, E., ... & Parren, P. W. (2013, November). Production of stable bispecific IgG1 by controlled Fab-arm exchange: scalability from bench to large-scale manufacturing by application of standard approaches. In MAbs (Vol. 5, No. 6, pp. 962-973). Taylor & Francis.); a heterodimeric protein form formed by combining structural calculations, Fc amino acid mutations, and different modes of action by the technician in Xencorto (Moore, G. L., Bautista, C., Pong, E., Nguyen, D. H. T., Jacinto, J., Eivazi, A., ... & Lazar, G. A. (2011, November). A novel bispecific antibody format enables simultaneous bivalent and monovalent co-engagement of distinct target antigens. In MAbs (Vol. 3, No. 6, pp. 546-557). Taylor & Francis.); a heterodimeric protein form obtained by the charge network-based Fc modification method of Alphamab Oncology (CN 201110459100.7); and other genetic engineering methods for forming heterodimeric functional proteins based on Fc amino acid changes or functional modification means. The Knob/Hole structure on the Fc variant fragments of the present disclosure means that the two Fc fragments are mutated respectively, and can be combined in a "Knob-into-hole" form after the mutations. Preferably, the Fc regions are subjected to site mutation modification using the "Knob-into-hole" model of Cater et al., so that the obtained first Fc variant and second Fc variant can be combined together in a "Knob-into-hole" form to form a heterodimer. The selection of particular immunoglobulin Fc regions from particular immunoglobulin classes and subclasses is within the range of those skilled in the art. The Fc regions of human antibodies IgG1, IgG2, IgG3 and IgG4 are preferred, and the Fc region of human antibody IgG1 is more preferred. Either of the first Fc variant or the second Fc variant is randomly selected for knob mutation and the other for hole mutation.

The term "effector function" herein refers to the activity of an antibody molecule, which is mediated by the binding of the domain of the antibody rather than the antigen binding domain, usually mediated by the binding of an effector molecule. Effector functions include complement-mediated effector functions, which are mediated by, for example, the binding of the C1 component of the complement to the antibody. The activation of a complement is important in the conditioning and lysis of cell pathogens. The activation of a complement also stimulates inflammatory response and can participate in autoimmune hypersensitivity response. Effector functions also include Fc receptor (FcR)-mediated effector functions, which can be triggered by the binding of the constant domain of the antibody to the Fc receptor (FcR). The binding of antibodies to Fc receptors on the cell surface triggers many important and diverse biological responses, including phagocytosis and destruction of antibody-coated particles, clearance of immune complexes, lysis of antibody-coated target cells by killer cells (called antibody-dependent cell-mediated cytotoxicity, or ADCC), release of inflammatory mediators, placental transfer, and control of immunoglobulin production. The effector functions of an antibody can be changed by, for example, enhancing or decreasing the affinity of the antibody to an effector molecule such as an Fc receptor or a complement component. Generally, the binding affinity will be changed by modifying the binding site of an effector molecule, and in this case, it is appropriate to locate the site of interest and modify at least part of the site in an appropriate manner. It is also assumed that changes in binding sites on an antibody against an effector molecule do not need to significantly change the overall binding affinity, but may change the geometry of the interaction, resulting in the invalid effector mechanism, as in unproductive binding. It is further assumed that effector functions can also be changed by modifying sites that are not directly involved in the binding of effector molecules but otherwise participate in the effector functions.

The term "conservative amino acid" herein generally refers to amino acids that belong to the same class or have similar characteristics (e.g., charge, side chain size, hydrophobicity, hydrophilicity, backbone conformation and rigidity). Exemplarily, the following amino acids in each group are each other's conservative amino acid residues, and a substitution of amino acid residues in the group is a substitution of conservative amino acids:
1) Alanine (A), Serine (S) and Threonine (T);
2) Aspartic acid (D) and Glutamic acid (E);
3) Asparagine (N) and Glutamine (Q);
4) Arginine (R), Lysine (K) and Histidine (H);
5) Isoleucine (I), Leucine (L), Methionine (M) and Valine (V); and
6) Phenylalanine (F), Tyrosine (Y) and Tryptophan (W).

The term "identity" herein can be calculated by the following method. To determine the percent "identity" of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position. The percent identity between two sequences will vary with the identical positions shared by the sequences, considering the number of gaps that need to be introduced to optimally align the two sequences and the length of each gap.

The sequence comparison and the calculation of percent identity between two sequences can be achieved using a mathematical algorithm. For example, the percent identity between two amino acid sequences is determined using the Needlema and Wunsch algorithm in the GAP program that has been integrated into the GCG software package (available at www.gcg.com) and using the Blossum 62 matrix or the PAM250 matrix, with a gap weight of 16, 14, 12, 10, 8, 6 or 4 and a length weight of 1, 2, 3, 4, 5 or 6. For another example, the percent identity between two nucleotide sequences is determined using the GAP program in the GCG software package (available at www.gcg.com) and using the NWSgapdna.CMP matrix, with a gap weight of 40, 50, 60, 70 or 80 and a length weight of 1, 2, 3, 4, 5 or 6. A particularly preferred parameter set (and one parameter set that should be used unless otherwise stated) is the Blossum62 scoring matrix with a gap penalty of 12, a gap extension penalty of 4, and a gap frameshift penalty of 5. The percent identity between two amino acid sequences or nucleotide sequences can also be determined using the PAM120 weighted remainder table, with a gap length penalty of 12 and a gap penalty of 4, and using the E. Meyers and W. Miller algorithm that has been incorporated into the ALIGN program (version 2.0).

Additionally or alternatively, the nucleic acid and protein sequences of the present disclosure can further be used as "query sequences" to perform searches against public databases, e.g., to identify other family member sequences or related sequences. For example, the NBLAST and XBLAST programs (version 2.0) of Altschul et al., (1990) J. Mol. Biol. 215:403-10 can be used to perform such searches. BLAST nucleotide searches can be performed with the NBLAST program, with score=100 and word length=12, to obtain the nucleotide sequences homologous to the nucleic acid molecules of the present disclosure. BLAST protein searches can be performed with the XBLAST program, with score=50 and word length=3, to obtain the amino acid sequences homologous to the protein molecules of the present disclosure. To obtain gapped alignment results for comparison purposes, gapped BLAST can be used as described in Altschul, S. F., Madden, T. L., Schäffer, A. A., Zhang, J., Zhang, Z., Miller, W., & Lipman, D. J. (1997). Gapped BLAST and PSI-BLAST: a new generation of protein database search programs. Nucleic acids research, 25 (17), 3389-3402. When BLAST and gapped BLAST programs are used, the default parameters of the corresponding programs (e.g., XBLAST and NBLAST) can be used. See www.ncbi.nlm.nih.gov.

The term "nucleic acid" herein includes any compound and/or substance comprising a polymer of nucleotides. Each nucleotide consists of a base, specifically a purine or pyrimidine base (i.e., cytosine (C), guanine (G), adenine (A), thymine (T) or uracil (U)), a sugar (i.e., deoxyribose or ribose) and a phosphate group. Generally, a nucleic acid molecule is described by a sequence of bases, whereby the bases represent the primary structure (linear structure) of the nucleic acid molecule. The sequence of bases is usually expressed as 5' to 3'. In this context, the term nucleic acid molecule encompasses deoxyribonucleic acid (DNA), including for example complementary DNA (cDNA) and genomic DNA, ribonucleic acid (RNA), especially messenger RNA (mRNA), synthetic forms of DNA or RNA, and a polymer comprising a mixture of two or more of these molecules. The nucleic acid molecule can be linear or circular. Furthermore, the term nucleic acid molecule includes both sense strand and antisense strand, as well as single-stranded form and double-stranded form. Furthermore, the nucleic acid molecules described herein may contain naturally occurring or non-naturally occurring nucleotides. Examples of the non-naturally occurring nucleotides include modified nucleotide bases with derivatized sugar or phosphate backbone linkages or chemically modified residues. The nucleic acid molecule also encompasses DNA and RNA molecules which are suitable as vectors for direct expression of the antibody of the present disclosure in vitro and/or in vivo, for example in a host or patient. Such DNA (e.g., cDNA) or RNA (e.g., mRNA) vectors may be unmodified or modified. For example, mRNA can be chemically modified to enhance the stability of the RNA vector and/or the expression of the encoded molecule, so that the mRNA can be injected into a subject to generate an antibody in vivo (see, for example, Stadler, C. R., Bähr-Mahmud, H., Celik, L., Hebich, B., Roth, A. S., Roth, R. P., ... & Sahin, U. (2017). Elimination of large tumors in mice by mRNA-encoded bispecific antibodies. Nature medicine, 23 (7), 815-817. or EP 2101823 B1).

The term "isolated" nucleic acid herein refers to a nucleic acid molecule that has been separated from components of the natural environment thereof. The isolated nucleic acid includes a nucleic acid molecule contained in a cell that normally contains the nucleic acid molecule, but which is present extrachromosomally or at a chromosomal location other than the natural chromosomal location thereof.

The term "vector" herein refers to a nucleic acid molecule capable of amplifying another nucleic acid to which it is linked. The term includes a vector having a self-replicating nucleic acid structure and a vector that is integrated into the genome of a host cell into which the vector has been introduced. Certain vectors can direct the expression of the nucleic acid to which they are operably linked. Such vectors are referred to herein as "expression vectors".

The term "host cell" herein refers to a cell into which a foreign nucleic acid is introduced, including the progeny of such a cell. The host cell includes a "transformant" and a "transformed cell" which include a primary transformed cell and the progeny derived therefrom, regardless of the number of passages. The progeny may not be identical to the parental cell in nucleic acid content, and may contain a mutation. The mutant progenies with the same function or biological activity as those screened or selected in the initially transformed cells are included herein.

The term "pharmaceutical composition" herein refers to a preparation that is present in a form which allows the active ingredients contained therein to be biologically effective and does not contain additional ingredients that would be unacceptably toxic to a subject to which the pharmaceutical composition is administered.

The term "pharmaceutically acceptable carrier" herein includes any and all solvents, dispersion media, coating materials, surfactants, antioxidants, preservatives (e.g., antibacterial agents and antifungal agents), isotonic agents, absorption delaying agents, salts, preservatives, drug stabilizers, binders, excipients, disintegrants, lubricants, sweeteners, flavoring agents, dyes, etc., and a combination thereof, which are known to those skilled in the art (see, for example, Remington's Pharmaceutical Sciences, 18th edition. MackPrinting Company, 1990, pages 1289-1329). Except in cases of incompatibility with the active ingredient, any conventional carrier is contemplated for use in a therapeutic or pharmaceutical composition.

The term "treatment" herein refers to surgical or therapeutic treatment, the purpose of which is to prevent and slow down (reduce) an undesired physiological change or pathology in a subject being treated, such as cancers and tumors. Beneficial or desired clinical results include, but are not limited to, alleviation of symptoms, diminishment of extent of disease, stabilized (*i.e.*, not worsening) state of disease, delay or slowing of disease progression, amelioration or palliation of the disease state and remission (whether partial response or complete response), whether detectable or undetectable. Subjects in need of treatment include subjects who already have a condition or a disease, subjects who are prone to a condition or a disease or subjects who intend to prevent a condition or a disease. When referring to terms such as slowing down, alleviation, diminishment, palliation and remission, the meaning of elimination, disappearance, non-occurrence, etc. is also included.

The term "subject" herein refers to an organism receiving treatment for a particular disease or condition as described herein. Exemplarily, a "subject" includes a mammal, such as a human, a primate (e.g., monkey) or a non-primate mammal, receiving treatment for a disease or a condition.

The term "effective amount" herein refers to an amount of a therapeutic agent effective to prevent or relieve a disease or a condition or the progression of the disease when administered alone or in combination with another therapeutic agent to a cell, tissue or subject. The "effective amount" also refers to an amount of a compound sufficient to relieve symptoms, for example, treat, cure, prevent or relieve the associated medical conditions, or to increase the rate of treating, curing, preventing or relieving these conditions. When an active ingredient is administered to an individual alone, a therapeutically effective dose refers to the ingredient alone. When a combination is used, a therapeutically effective dose refers to the combined amounts of the active ingredients that produce a therapeutic effect, whether administered in combination, sequentially or simultaneously.

The term "cancer" herein refers to or describes the physiological condition in mammals that is typically characterized by unregulated cell growth. Both benign and malignant cancers are included in this definition. The term "tumor" or "neoplasm" herein refers to all neoplastic cell growth and proliferation, whether malignant or benign, and to all pre-cancerous and cancerous cells and tissues. The terms "cancer" and "tumor" are not mutually exclusive when referred to herein.

The term "EC50" herein refers to the half-maximal effective concentration, which includes the concentration of an antibody that induces a response halfway between baseline and maximum after a specified exposure time. EC50 essentially represents the concentration of an antibody at which 50% of the maximal effect thereof is observed and which can be measured by methods known in the art.

### Detailed Description of Embodiments

GPRC5D is G protein coupled receptor family C group 5 member D, which is an orphan receptor and is a seven-transmembrane protein. GPRC5D is a new specific target for multiple myeloma after BCMA. It is found in tissue expression profiling studies that GPRC5D is specifically highly expressed in plasma cells of multiple myeloma, while has a low expression in normal tissues, which is limited to hair follicle regions with immune privilege. Moreover, the expression of GPRC5D does not coincide with that of BCMA. For tumor recurrence models with BCMA loss, GPRC5D CAR-T still has a therapeutic effect, overcoming tumor escape. CD3 molecule is an important marker of T cells and is a portion of T cell receptor (TCR), which can induce T cell activation and is related to tumor immunity.

The present disclosure provides a new multi-specific antigen binding molecule that target tumor antigens BCMA, GPRC5D and T cell (CD3), in which the two different targets of multiple myeloma are bound while the T cell is induced to participate in tumor killing to enhance a tumor killing effect, thereby providing a new tumor treatment method.

The present disclosure further provides a nucleic acid fragment encoding a multi-specific antibody or an antigen binding fragment that specifically bind to B cell maturation antigen (BCMA), GPRC5D and T cell, a vector, a host cell, an immune effector cell, a preparation method, a pharmaceutical composition, a pharmaceutical use, and a method for treating tumors or cancers (e.g., B cell lymphoma or multiple myeloma).

In a first aspect, the present disclosure provides a multi-specific binding molecule, comprising:
(a) a first antigen binding molecule (ABM1) that specifically binds to a first tumor-associated antigen (TAA1);
(b) a second antigen binding molecule (ABM2) that specifically binds to a second tumor-associated antigen (TAA2); and
(c) a third antigen binding molecule (ABM3) that specifically binds to an antigen expressed on a human immune cell.

In some embodiments, the TAA1 and/or the TAA2 are expressed on a cancerous B cell.

In some embodiments, the cancerous B cell is a plasma cell-derived or B cell-derived.

In some embodiments, the immune cell is selected from a T cell, a NK cell or a macrophage.

In some embodiments, the TAA1 and TAA2 are each independently selected from BCMA and GPRC5D, and/or the antigen expressed on the immune cell is CD3.

In some embodiments, the ABM1, ABM2 and ABM3 may be each independently selected from an antibody, an antibody fragment, F(ab')₂, Fab', Fab, Fv, scFv, a nanobody or VHH.

In some embodiments, the multi-specific binding molecule comprises four polypeptide chains: a first heavy chain, a first light chain, a second heavy chain and a second light chain.

In some specific embodiments, wherein (1) the first heavy chain and the second heavy chain are the same and have the structure as shown below:
(a) VHₐ₋CH1-L1-VHH_{b}-Fc-L2-VH_{CD3}-L3-VL_{CD3};
(b) VHₐ-CH1-L1-VH_{CD3}-L2-VL_{CD3}-Fc-L3-VHH_{b};
(c) VHH_{b}-L1-VHₐ-CH1-L2-VH_{CD3}-L3-VL_{CD3}-Fc;
(d) VHₐ-CH1-L1-VHH_{b}-L2-VH_{CD3}-L3-VL_{CD3}-Fc; or
(e) VHₐCH1-L1-VH_{b}-L2-VL_{b}-Fc-L3-VH_{CD3}-L4-VL_{CD3};

and/or, (2) the first light chain and the second light chain are the same and have the structure as shown below: VLₐ-CL;
wherein VHₐ and VLₐ are a heavy chain variable region and a light chain variable region that specifically bind to GPRC5D, respectively; VHH_{b} is a nanobody that specifically binds to BCMA, VH_{b} and VL_{b} are a heavy chain variable region and a light chain variable region that specifically bind to BCMA, respectively; VH_{CD3} and VL_{CD3} are a heavy chain variable region and a light chain variable region that specifically bind to CD3, respectively; Fc is a Fc region of any antibody; and L1, L2, L3 and L4 are the same or different linkers, respectively.

In some specific embodiments, wherein (1) the first heavy chain and the second heavy chain are different and have the structure as shown below:
(a) a first heavy chain VHₐ-CH1-L1-VHH_{b}-Fc1 and a second heavy chain VHₐ-CH1-L2-VH_{CD3}-L3-VL_{CD3}-Fc2 ;
(b) a first heavy chain VHH_{b}-L1-VHₐ-CH1-FC1 and a second heavy chain VHₐ-CH1-L2- VH_{CD3}-L3-VL_{CD3}-Fc2;
(c) a first heavy chain VHₐ₋CH1-L1-VH_{b}-L2-VL_{b}-Fc1 and a second heavy chain VHₐ-CH1-L3-VH_{b}-L4-VL_{b}-Fc2- L5-VH_{CD3}-L6-VL_{CD3};
(d) a first heavy chain VHₐ-CH1-L1-VHH_{b}-Fc1 and a second heavy chain VHₐ-CH1-L2-VHH_{b}-Fc2-L3-VH_{CD3}-L4- VL_{CD3}; or
(e) a first heavy chain VHH_{b}-L1-VHₐ-CH1-Fc1 and a second heavy chain VHH_{b}-L2-VHₐ-CH1-Fc2-L3-VH_{CD3}-L4- VL_{CD3};

optionally, the first heavy chain further has a Flag-tag at the carboxyl terminus and/or the second heavy chain further has a His-tag at the carboxyl terminus in the above-mentioned (a)-(e);
and, (2) the first light chain and the second light chain are the same and have the structure as shown below: VLₐ-CL;
wherein VHₐ and VLₐ are a heavy chain variable region and a light chain variable region that specifically bind to GPRC5D, respectively; VHH_{b} is a nanobody that specifically binds to BCMA, VH_{b} and VL_{b} are a heavy chain variable region and a light chain variable region that specifically bind to BCMA, respectively; VH_{CD3} and VL_{CD3} are a heavy chain variable region and a light chain variable region that specifically bind to CD3, respectively; Fc1 and Fc2 are each a Fc region of any antibody; and L1, L2, L3, L4, L5 and L6 are the same or different linkers, respectively.

In some embodiments, the multi-specific binding molecule comprises three polypeptide chains: a first heavy chain, a second heavy chain and a light chain, wherein (1) the first heavy chain and the second heavy chain have the structure as shown below:
(a) a first heavy chain VHH_{b}-L1-VHH_{b}-Fc1 and a second heavy chain VHₐ-CH1-L2- VH_{CD3}-L3-VL_{CD3}-Fc2;
(b) a first heavy chain VHH_{b}-L1-VH_{CD3}-L2-VL_{CD3}-Fc1 and a second heavy chain VHₐ-CH1-Fc2; or
(c) a first heavy chain VHH_{b}-Fc1 and a second heavy chain VHₐ-CH1-L1-VH_{CD3}-L2-VL_{CD3}-Fc2;

optionally, the first heavy chain further has a Flag-tag at the carboxyl terminus and/or the second heavy chain further has a His-tag at the carboxyl terminus in the above-mentioned (a)-(c);
and, (2) the light chain has the structure as shown below: VLₐ-CL;
wherein VHₐ and VLₐ are a heavy chain variable region and a light chain variable region that specifically bind to GPRC5D, respectively; VHH_{b} is a nanobody that specifically binds to BCMA, VH_{b} and VL_{b} are a heavy chain variable region and a light chain variable region that specifically bind to BCMA, respectively; VH_{CD3} and VL_{CD3} are a heavy chain variable region and a light chain variable region that specifically bind to CD3, respectively; and L1, L2 and L3 are the same or different linkers, respectively.

In some embodiments, Fc1 and Fc2 of the multi-specific binding molecule are different, wherein Fc1 is knob-Fc and Fc2 is hole-Fc; or, Fc1 is hole-Fc, and Fc2 is knob-Fc; and preferably, Fc1 and Fc2 are Fc regions of IgG1 or IgG4.

In some specific embodiments, wherein the knob-Fc comprises a T366W mutation, and/or the hole-Fc comprises a T366S, L368A and/or Y407V mutation.

In some embodiments, the ABM3 in the multi-specific binding molecule is an anti-CD3 antibody or an antigen binding fragment thereof, and comprises a light chain variable region and a heavy chain variable region: (a) the light chain variable region comprises LCDR1, LCDR2 and LCDR3, and the LCDR1, LCDR2 and LCDR3 have amino acid sequences as shown in SEQ ID NOs: 54-56, or having 1, 2, 3 or more amino acid insertions, deletions and/or substitutions compared to SEQ ID NOs: 54-56; and (b) the heavy chain variable region comprises HCDR1, HCDR2 and HCDR3, and the HCDR1, HCDR2 and HCDR3 have amino acid sequences as shown in SEQ ID NOs: 51-53, or having 1, 2, 3 or more amino acid insertions, deletions and/or substitutions compared to SEQ ID NOs: 51-53.

In some specific embodiments, the light chain variable region comprises an amino acid sequence as shown in SEQ ID NO: 10 or comprises an amino acid sequence having a 90%, 95%, 96%, 97%, 98%, 99% or more identity to SEQ ID NO: 10, and/or the heavy chain variable region comprises an amino acid sequence as shown in SEQ ID NO: 9 or comprises an amino acid sequence having a 90%, 95%, 96%, 97%, 98%, 99% or more identity to SEQ ID NO: 9.

In some specific embodiments, the ABM3 is scFv.

In some embodiments, the TAA1 and TAA2 in the multi-specific binding molecule are selected from the following group: (a) TAA1 is BCMA, and TAA2 is GPRC5D; or (b) TAA1 is GPRC5D, and TAA2 is BCMA.

In some specific embodiments, the antigen binding molecule that specifically binds to GPRC5D in the multi-specific binding molecule comprises a light chain variable region and a heavy chain variable region of an antibody, wherein (a) the light chain variable region comprises LCDR1, LCDR2 and LCDR3, the LCDR1, LCDR2 and LCDR3 have amino acid sequences as shown in SEQ ID NOs: 42-44, or having 1, 2, 3 or more amino acid insertions, deletions and/or substitutions compared to SEQ ID NOs: 42-44, the heavy chain variable region comprises HCDR1, HCDR2 and HCDR3, and the HCDR1, HCDR2 and HCDR3 have amino acid sequences as shown in SEQ ID NOs: 39-41, or having 1, 2, 3 or more amino acid insertions, deletions and/or substitutions compared to SEQ ID NOs: 39-41; or (b) the light chain variable region comprises LCDR1, LCDR2 and LCDR3, the LCDR1, LCDR2 and LCDR3 have amino acid sequences as shown in SEQ ID NOs: 48-50, or having 1, 2, 3 or more amino acid insertions, deletions and/or substitutions compared to SEQ ID NOs: 48-50, the heavy chain variable region comprises HCDR1, HCDR2 and HCDR3, and the HCDR1, HCDR2 and HCDR3 have amino acid sequences as shown in SEQ ID NOs: 45-47, or having 1, 2, 3 or more amino acid insertions, deletions and/or substitutions compared to SEQ ID NOs: 45-47.

In some specific embodiments, wherein:
(a) the light chain variable region comprises an amino acid sequence as shown in SEQ ID NO: 6 or comprises an amino acid sequence having a 90%, 95%, 96%, 97%, 98%, 99% or more identity to SEQ ID NO: 6, and/or the heavy chain variable region comprises an amino acid sequence as shown in SEQ ID NO: 5 or comprises an amino acid sequence having a 90%, 95%, 96%, 97%, 98%, 99% or more identity to SEQ ID NO: 5; or
(b) the light chain variable region comprises an amino acid sequence as shown in SEQ ID NO: 8 or comprises an amino acid sequence having a 90%, 95%, 96%, 97%, 98%, 99% or more identity to SEQ ID NO: 8, and/or the heavy chain variable region comprises an amino acid sequence as shown in SEQ ID NO: 7 or comprises an amino acid sequence having a 90%, 95%, 96%, 97%, 98%, 99% or more identity to SEQ ID NO: 7.

In some specific embodiments, the antigen binding molecule that specifically binds to GPRC5D in the multi-specific binding molecule is Fab.

In some specific embodiments, the antigen binding molecule that specifically binds to BCMA in the multi-specific binding molecule comprises a heavy chain variable region of an antibody, the heavy chain variable region comprises HCDR1, HCDR2 and HCDR3, and the HCDR1, HCDR2 and HCDR3 have amino acid sequences as shown in SEQ ID NOs: 27-29 or as shown in SEQ ID NOs: 30-32, or having 1, 2, 3 or more amino acid insertions, deletions and/or substitutions compared to SEQ ID NOs: 27-29 or SEQ ID NOs: 30-32.

In some specific embodiments, the heavy chain variable region comprises an amino acid sequence as shown in SEQ ID NO: 1 or SEQ ID NO: 2, or comprises an amino acid sequence having a 90%, 95%, 96%, 97%, 98%, 99% or more identity to SEQ ID NO: 1 or SEQ ID NO: 2.

In some specific embodiments, the antigen binding molecule that specifically binds to BCMA is VHH.

In some specific embodiments, the antigen binding molecule that specifically binds to BCMA in the multi-specific binding molecule comprises a light chain variable region and a heavy chain variable region of an antibody: (a) the light chain variable region comprises LCDR1, LCDR2 and LCDR3, and the LCDR1, LCDR2 and LCDR3 have amino acid sequences as shown in SEQ ID NOs: 36-38, or having 1, 2, 3 or more amino acid insertions, deletions and/or substitutions compared to SEQ ID NOs: 36-38; and (b) the heavy chain variable region comprises HCDR1, HCDR2 and HCDR3, and the HCDR1, HCDR2 and HCDR3 have amino acid sequences as shown in SEQ ID NOs: 33-35, or having 1, 2, 3 or more amino acid insertions, deletions and/or substitutions compared to SEQ ID NOs: 33-35.

In some specific embodiments, the light chain variable region comprises an amino acid sequence as shown in SEQ ID NO: 4 or comprises an amino acid sequence having a 90%, 95%, 96%, 97%, 98%, 99% or more identity to SEQ ID NO: 4, and/or the heavy chain variable region comprises an amino acid sequence as shown in SEQ ID NO: 3 or comprises an amino acid sequence having a 90%, 95%, 96%, 97%, 98%, 99% or more identity to SEQ ID NO: 3.

In some specific embodiments, the antigen binding molecule that specifically binds to BCMA is scFv.

In some embodiments, the ABM1, ABM2 and/or ABM3 is chimeric, humanized or fully humanized.

In some embodiments, the multi-specific binding molecule further comprises an Fc fragment of human IgG1, IgG2, IgG3 or IgG4; and preferably, the multi-specific binding molecule comprises an Fc fragment of human IgG1 or human IgG4.

In some specific embodiments, the Fc fragment of the multi-specific binding molecule has an amino acid mutation that changes effector functions; preferably, the amino acid mutation that changes effector functions comprises an S228P, F234A, L234A and/or L235A mutation; and more preferably, the amino acid mutation that changes effector functions comprises: (a) a L234A and/or L235A mutation of IgG1 Fc; and/or (b) an S228P, F234A and/or L235A mutation of IgG4 Fc.

In some embodiments, the Fc fragment of the multi-specific binding molecule comprises the same or different Fc1 and Fc2.

In some specific embodiments, the different Fc1 and Fc2 in the multi-specific binding molecule have amino acid mutations that promote heterodimerization; preferably, the amino acid mutations that promote heterodimerization comprise a Knob-into-hole (KIH) mutation; and more preferably, the Knob-into-hole mutation comprises: (a) a T366W mutation in any Fc region; and (b) a T366S, L368A and/or Y407V mutation in another Fc region.

In some specific embodiments, the Fc1 further comprises a His-tag, and the Fc2 further comprises a Flag-tag; or the Fc1 further comprises a Flag-tag, and the Fc2 further comprises a His-tag.

In some embodiments, the multi-specific binding molecule is trivalent, tetravalent, pentavalent or hexavalent.

In a second aspect, the present disclosure provides an isolated nucleic acid, encoding the aforementioned multi-specific binding molecule.

In a third aspect, the present disclosure provides a recombinant vector, comprising the aforementioned isolated nucleic acid.

In a fourth aspect, the present disclosure provides a host cell, comprising the aforementioned isolated nucleic acid or the aforementioned recombinant vector; preferably, the host cell is a eukaryotic cell or a prokaryotic cell; more preferably, the host cell is derived from a mammalian cell, a yeast cell, an insect cell, Escherichia coli and/or Bacillus subtilis; and more preferably, the host cell is Expi293 cell.

In a fifth aspect, the present disclosure provides a method for preparing a multi-specific binding molecule, comprising: culturing the aforementioned host cell under appropriate conditions, and isolating the multi-specific binding molecule.

In a sixth aspect, the present disclosure provides a pharmaceutical composition, comprising the aforementioned multi-specific binding molecule, the aforementioned isolated nucleic acid, the aforementioned recombinant vector, the aforementioned host cell, or a product prepared according to the aforementioned method, and one or more pharmaceutically acceptable carriers, diluents, buffers or excipients; more preferably, the pharmaceutical composition further comprises an additional cancer therapeutic agent.

In a seventh aspect, the present disclosure provides a method for activating T cells, comprising: administering to a subject a therapeutically effective amount of the aforementioned multi-specific binding molecule or pharmaceutical composition.

In an eighth aspect, the present disclosure provides the aforementioned multi-specific binding molecule, the aforementioned isolated nucleic acid, the aforementioned host cell, a product prepared by the aforementioned method or the aforementioned pharmaceutical composition, for use in the preparation of a drug for preventing and/or treating cancers, or for use in the treatment and/or prevention of cancers.

In preferred embodiments, the cancer is B-cell lymphoma; and more preferably, the B-cell lymphoma is multiple myeloma.

In a ninth aspect, the present disclosure provides a method for treating and/or preventing cancers, comprising: administering to a subject a therapeutically effective amount of the aforementioned multi-specific binding molecule, the aforementioned isolated nucleic acid, the aforementioned host cell, a product prepared by the aforementioned method or the aforementioned pharmaceutical composition.

In preferred embodiments, the cancer is B-cell lymphoma; and more preferably, the B-cell lymphoma is multiple myeloma.

### Examples

The present disclosure will be further described below in conjunction with specific examples, and the advantages and characteristics of the present disclosure will become clearer along with the description. If specific conditions are not specified in the examples, conventional conditions or conditions recommended by a manufacturer are followed. The reagents or instruments used therein for which manufacturers are not specified are all conventional products that are commercially available.

The examples of the present disclosure are merely exemplary, and do not limit the scope of the present disclosure in any way. Those skilled in the art should understand that the details and forms of the technical solutions of the present disclosure can be modified or replaced without departing from the spirit and scope of the present disclosure, but these modifications and replacements all fall within the scope of protection of the present disclosure.

### Example 1 Construction of anti-BCMA-GPRC5D-CD3 trispecific antibody molecule

After a series of experiments such as animal immunization, sequencing and humanization, better humanized anti-BCMA specific antibodies (BCMA-VHH01, BCMA-VHH02 and BCMA-Hab01), humanized anti-GPRC5D specific antibodies (GPRC5D-Hab01 and GPRC5D-Hab02) and a humanized anti-CD3 specific antibody (CD3-Hab01) were obtained, and then these specific antibodies were assembled into an anti-BCMA-GPRC5D-CD3 trispecific antibody, wherein the assembling method could be in a ratio of 2 : 2 : 2 (BCMA:GPRC5D:CD3) (exemplarily as shown in A-G in FIG. 1), or 1 : 2 : 1 (BCMA:GPRC5D:CD3 or GPRC5D:BCMA:CD3) (exemplarily as shown in H-J in FIG. 1), or 1 : 1 : 1 (BCMA:GPRC5D:CD3) (exemplarily as shown in N or O in FIG. 1), or 2 : 2 : 1 (BCMA:GPRC5D:CD3) (exemplarily as shown in K-M in FIG. 1), the antibody fragment was scFv, Fab or VHH, the heavy chain constant region subtype was IgG1 or IgG4, and if the molecule was of asymmetric structure, mispairing was reduced by "Knob in Hole". Finally, the fragments were linked via linkers and constructed on a PTT5 expression vector to obtain a target plasmid. The schematic diagrams of the trispecific antibody molecules were as shown in FIG. 1, the molecular composition of each trispecific antibody was as shown in Table 1, the amino acid sequences of the antibody molecules were as shown in Table 2, and the CDRs of the main antibody variable regions determined by Kabat numbering system were as shown in Table 3. In this example, the positive control antibodies used were a bispecific antibody REGN5459 (WO 2020018820 A1, targeting antigen BCMA and CD3 receptor expressed on T cell surface) from Regeneron and a bispecific antibody JNJ7564 (WO 2019220368 A1, targeting antigen GPRC5D and CD3 receptor expressed on T cell surface) from Johnson & Johnson, respectively, and the negative control antibody was antiFITC×CD3-Hab01 (prepared internally). The structures of the positive and negative control antibodies were as shown in P-R in FIG. 1, in which the Fc regions of REGN5459 and JNJ7564 comprised mutations that prevent homodimerization.

**Table 1 Structural compositions of trispecific antibody molecules**

| **Designation of antibody** | **BCMA-targeting moiety** | **GPRC5D-targeting moiety** | **CD3-targeting moiety** | **Heavy chain constant region subtype** |
|---|---|---|---|---|
| Tri-17, Tri-29, Tri-32, Tri-35 | BCMA-VHH01 | GPRC5D-Hab01 | CD3-Hab01 | IgG4 Fc |
| Tri-38, Tri-41 | | | | IgG1 Fc Knob in Hole |
| Tri-46, Tri-49, Tri-52, Tri-56, Tri-63, Tri-64 | BCMA-VHH02 | | | IgG4 Fc Knob in Hole |
| Tri-55, Tri-59 | | | | IgG4 Fc |
| Tri-60 | BCMA-Hab01 | | | IgG4 Fe |
| Tri-62 | | | | IgG4 Fc Knob in Hole |
| Tri-65, Tri-66 | BCMA-VHH02 | GPRC5D-Hab02 | | IgG4 Fc Knob in Hole |

**Table 2 Amino acid sequences of antibody molecules**

| **Designation of sequence** | **Amino acid sequence** | **Sequence number** |
|---|---|---|
| BCMA-VHH01 | EVQLVESGGGLVQPGGSLRLSCSASGFTLDYYMIGWFRQAPG KEREGLSSISPADGSTYYADSVKGRFTISRDSSKNTVYLQMNS LRAEDTAVYYCAAGNEATISWGFGPWEYDYWGQGTMVTVSS | SEQ ID NO.1 |
| BCMA-VHH02 | EVQLVESGGGLVQPGGSLRLSCAASESISSIHIMAWYRQAPGK QRELVAGIRNDGSTVYVDSVKGRFTISRDNAKNSLYLQMNSL RAEDTAVYYCNADQGFGSYSEWERRSRWGQGTTVTVSS | SEQ ID NO.2 |
| BCMA-Hab01 VH | EGQMVQSGAEVKKPGSSVKVSCKASGYMFTDHTFHWVRQA PGQGLEWMGYIYPRDQNTKYNEKFKGRVTITADKSTSTAYME LSSLRSEDTAVYYCARAGYYGSSHAMDYWGQGTTVTVSS | SEQ ID NO.3 |
| BCMA-Hab01 VL | DIQMTQSPSSLSASVGDRVTITCKASQDVSTAVAWYQQKPGKS PRLLIYSASSRYTGVPSRFSGSGSGTDFTFTISSLQPEDIATYYC QQLYSTPWTFGGGTKVEIK | SEQ ID NO.4 |
| GPRC5D-Hab01 VH | EVQLVQSGAEVKKPGSSVKVSCKASGYTFTSYGISWVRQAPG QGLEWMGEIYPRSGNTYYNEKFKGRVTLTADKSTSTAYMELS SLRSEDTAVYYCARGRAYGRNYPMDYWGQGTTVTVSS | SEQ ID NO.5 |
| GPRC5D-Hab01 VL | EIVLTQSPATLSLSPGERATLSCRASQSIGTSIHWYQQKPGQSPR LLIKYASESISGIPARFSGSGSGTDFTLTISSLEPEDFAVYYCQQS NSWPYTFGGGTKVEIK | SEQ ID NO.6 |
| GPRC5D-Hab02 VH | EVQLVQSGAEVKKPGASVKVSCKTSGYIFTNFGITWVRQAPG QRLEWMGENYPRRINTYYNEKFKGRVTITADKSASTAYMELS SLRSEDTAVYYCSRKDIFGLSYALDYWGQGTTVTVSS | SEQ ID NO.7 |
| GPRC5D-Hab02 VL | DIQMTQSPSSLSASVGDRVTITCKASQNVGTNVAWYQHKPGK SPKALIYSASYQYSGVPSRFSGSGSGTDFTLTISSLQPEDVATYI CQQYKNYPYTFGGGTKVEIK | SEQ ID NO.8 |
| CD3-Hab01 VH | EVQLVESGGGVVQPGRSLRLSCAASGFTFTDYYMSWVRQAP GKGLEWVAMSRNKAKGHTIEYSSSVKGRFTISRDNSKNTLYL QMNSLRAEDTAVYYCARDTYESYDGYFDVWGQGTTVTVSS | SEQ ID NO.9 |
| CD3-Hab01 VL | DIVMTQSPDSLAVSLGERATINCKSSQSLFNSRSRKNYLAWYQ QKPGQTPKLLIYWASIRESGVPDRFSGSGSGTDFTLTISSLQAE DVAVYYCKQSYYLYTFGQGTKLEIK | SEQ ID NO.10 |
| IgG1 Fc_Knob | EPKSSDKTHTCPPCPAPEAAGGPSVFLFPPKPKDTLMISRTPEV TCVVVDVSHEDPEVKFNWYVDGVEVHNAKTKPREEQYNST YRVVSVLTVLHQDWLNGKEYKCKVSNKALPAPIEKTISKAKG QPREPQVYTLPPSREEMTKNQVSLWCLVKGFYPSDIAVEWES NGQPENNYKTTPPVLDSDGSFFLYSKLTVDKSRWQQGNVFSC SVMHEALHNHYTQKSLSLSPG | SEQ ID NO.11 |
| IgG1 Fc_Hole | EPKSSDKTHTCPPCPAPEAAGGPSVFLFPPKPKDTLMISRTPEV TCVVVDVSHEDPEVKFNWYVDGVEVHNAKTKPREEQYNST YRVVSVLTVLHQDWLNGKEYKCKVSNKALPAPIEKTISKAKG QPREPQVYTLPPSREEMTKNQVSLSCAVKGFYPSDIAVEWESN GQPENNYKTTPPVLDSDGSFFLVSKLTVDKSRWQQGNVFSCS VMHEALHNHYTQKSLSLSPG | SEQ ID NO.12 |
| IgG4 Fc | ESKYGPPCPPCPAPEAAGGPSVFLFPPKPKDTLMISRTPEVTCV VVDVSQEDPEVQFNWYVDGVEVHNAKTKPREEQFNSTYRVV SVLTVLHQDWLNGKEYKCKVSNKGLPSSIEKTISKAKGQPREP QVYTLPPSQEEMTKNQVSLTCLVKGFYPSDIAVEWESNGQPEN NYKTTPPVLDSDGSFFLYSRLTVDKSRWQEGNVFSCSVMHEA LHNHYTQKSLSLSLGK | SEQ ID NO.13 |
| IgG4 Fc_Knob | ESKYGPPCPPCPAPEAAGGPSVFLFPPKPKDTLMISRTPEVTCV VVDVSQEDPEVQFNWYVDGVEVHNAKTKPREEQFNSTYRVV SVLTVLHQDWLNGKEYKCKVSNKGLPSSIEKTISKAKGQPREP QVYTLPPSQEEMTKNQVSLWCLVKGFYPSDIAVEWESNGQPE NNYKTTPPVLDSDGSFFLYSRLTVDKSRWQEGNVFSCSVMHE ALHNHYTQKSLSLSLG | SEQ ID NO.14 |
| IgG4 Fc_Hole | ESKYGPPCPPCPAPEAAGGPSVFLFPPKPKDTLMISRTPEVTCV VVDVSQEDPEVQFNWYVDGVEVHNAKTKPREEQFNSTYRVV SVLTVLHQDWLNGKEYKCKVSNKGLPSSIEKTISKAKGQPREP QVYTLPPSQEEMTKNQVSLSCAVKGFYPSDIAVEWESNGQPE NNYKTTPPVLDSDGSFFLVSRLTVDKSRWQEGNVFSCSVMHE ALHNHYTQKSLSLSLG | SEQ ID NO.15 |
| IgG1_CH1 | ASTKGPSVFPLAPSSKSTSGGTAALGCLVKDYFPEPVTVSWNS GALTSGVHTFPAVLQSSGLYSLSSVVTVPSSSLGTQTYICNVNH KPSNTKVDKKV | SEQ ID NO.16 |
| IgG4_CH1 | ASTKGPSVFPLAPCSRSTSESTAALGCLVKDYFPEPVTVSWNS GALTSGVHTFPAVLQSSGLYSLSSVVTVPSSSLGTKTYTCNVD HKPSNTKVDKRV | SEQ ID NO.17 |
| REGN5459-anti-BCMA heavy chain | EVQLVESGGGLVQPGGSLRLSCAASGFTFSNFWMTWVRQAP GKGLEWVANMNQDGSEKYYVDSVKGRFTISRDNAKSSLYLQ MNSLRAEDTAVYYCARDREYCISTSCYDDFDYWGQGTLVTVS SASTKGPSVFPLAPCSRSTSESTAALGCLVKDYFPEPVTVSWN SGALTSGVHTFPAVLQSSGLYSLSSVVTVPSSSLGTKTYTCNVD HKPSNTKVDKRVESKYGPPCPPCPAPPVAGPSVFLFPPKPKDTL MISRTPEVTCVVVDVSQEDPEVQFNWYVDGVEVHNAKTKPR EEQFNSTYRVVSVLTVLHQDWLNGKEYKCKVSNKGLPSSIEK TISKAKGQPREPQVYTLPPSQEEMTKNQVSLTCLVKGFYPSDI AVEWESNGQPENNYKTTPPVLDSDGSFFLYSRLTVDKSRWQE GNVFSCSVMHEALHNHYTQKSLSLSLGK | SEQ ID NO.18 |
| REGN5459-anti-CD3 heavy chain | EVQLVESGGGLVQPGRSLRLSCAASGFTFDDYSMHWVRQAP GKGLEWVSGISWNSGSIGYADSVKGRFTISRDNAKNSLYLQM NSLRAEDTALYYCAKYGSGYGKFYYYGMDVWGQGTTVTVS SASTKGPSVFPLAPCSRSTSESTAALGCLVKDYFPEPVTVSWN SGALTSGVHTFPAVLQSSGLYSLSSVVTVPSSSLGTKTYTCNVD HKPSNTKVDKRVESKYGPPCPPCPAPPVAGPSVFLFPPKPKDTL MISRTPEVTCVVVDVSQEDPEVQFNWYVDGVEVHNAKTKPR EEQFNSTYRVVSVLTVLHQDWLNGKEYKCKVSNKGLPSSIEK TISKAKGQPREPQVYTLPPSQEEMTKNQVSLTCLVKGFYPSDIA VEWESNGQPENNYKTTPPVLDSDGSFFLYSRLTVDKSRWQEG NVFSCSVMHEALHNRFTQKSLSLSLGK | SEQ ID NO.19 |
| REGN5459-common light chain | DIQMTQSPSSLSASVGDRVTITCRASQSISSYLNWYQQKPGKA PKLLIYAASSLQSGVPSRFSGSGSGTDFTLTISSLQPEDFATYYC QQSYSTPPITFGQGTRLEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | SEQ ID NO.20 |
| JNJ7564-anti-GPRC5D heavy chain | QVQLVQSGAEVKKPGASVKVSCKASGYSFTGYTMNWVRQA PGQGLEWMGLINPYNSDTNYAQKLQGRVTMTTDTSTSTAYM ELRSLRSDDTAVYYCARVALRVALDYWGQGTLVTVSSASTKG PSVFPLAPCSRSTSESTAALGCLVKDYFPEPVTVSWNSGALTSG VHTFPAVLQSSGLYSLSSVVTVPSSSLGTKTYTCNVDHKPSNT KVDKRVESKYGPPCPPCPAPEAAGGPSVFLFPPKPKDTLMISRT PEVTCVVVDVSQEDPEVQFNWYVDGVEVHNAKTKPREEQFN STYRVVSVLTVLHQDWLNGKEYKCKVSNKGLPSSIEKTISKA | SEQ ID NO.21 |
| | KGQPREPQVYTLPPSQEEMTKNQVSLTCLVKGFYPSDIAVEWE SNGQPENNYKTTPPVLDSDGSFFLYSRLTVDKSRWQEGNVFSC SVMHEALHNHYTQKSLSLSLGK | |
| JNJ7564-anti-GPRC5D light chain | DIQMTQSPSSLSASVGDRVTITCKASQNVATHVGWYQQKPGK APKRLIYSASYRYSGVPSRFSGSGSGTEFTLTISNLQPEDFATYY CQQYNRYPYTFGQGTKLEIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC | SEQ ID NO.22 |
| JNJ7564-anti-CD3 heavy chain | EVQLVESGGGLVQPGGSLRLSCAASGFTFNTYAMNWVRQAP GKGLEWVARIRSKYNNYATYYAASVKGRFTISRDDSKNSLYL QMNSLKTEDTAVYYCARHGNFGNSYVSWFAYWGQGTLVTVS SASTKGPSVFPLAPCSRSTSESTAALGCLVKDYFPEPVTVSWN SGALTSGVHTFPAVLQSSGLYSLSSVVTVPSSSLGTKTYTCNVD HKPSNTKVDKRVESKYGPPCPPCPAPEAAGGPSVFLFPPKPKD TLMISRTPEVTCVVVDVSQEDPEVQFNWYVDGVEVHNAKTK PREEQFNSTYRVVSVLTVLHQDWLNGKEYKCKVSNKGLPSSI EKTISKAKGQPREPQVYTLPPSQEEMTKNQVSLTCLVKGFYPS DIAVEWESNGQPENNYKTTPPVLDSDGSFLLYSKLTVDKSRW QEGNVFSCSVMHEALHNHYTQKSLSLSLGK | SEQ ID NO.23 |
| JNJ7564-anti-CD3 light chain | QTVVTQEPSLTVSPGGTVTLTCRSSTGAVTTSNYANWVQQKP GQAPRGLIGGTNKRAPGTPARFSGSLLGGKAALTLSGVQPEDE AEYYCALWYSNLWVFGGGTKLTVLGQPKAAPSVTLFPPSSEE LQANKATLVCLISDFYPGAVTVAWKADSSPVKAGVETTTPSKQ SNNKYAASSYLSLTPEQWKSHRSYSCQVTHEGSTVEKTVAPTE CS | SEQ ID NO.24 |
| antiFITC×C D3-Hab01 heavy chain | EVKLDETGGGLVQPGRPMKLSCVASGFTFSDYWMNWVRQSP EKGLEWVAQIRNKPYNYETYYSDSVKGRFTISRDDSKSSVYL QMNNLRVEDMGIYYCTGSYYGMDYWGQGTSVTVSSASTKG PSVFPLAPCSRSTSESTAALGCLVKDYFPEPVTVSWNSGALTSG VHTFPAVLQSSGLYSLSSVVTVPSSSLGTKTYTCNVDHKPSNT KVDKRVGGGGSGGGGSGGGGSEVQLVESGGGVVQPGRSLRL SCAASGFTFTDYYMSWVRQAPGKGLEWVAMSRNKAKGHTIE YSSSVKGRFTISRDNSKNTLYLQMNSLRAEDTAVYYCARDTY ESYDGYFDVWGQGTTVTVSSGGGGSGGGGSGGGGSDIVMTQ SPDSLAVSLGERATINCKSSQSLFNSRSRKNYLAWYQQKPGQT PKLLIYWASIRESGVPDRFSGSGSGTDFTLTISSLQAEDVAVYY CKQSYYLYTFGQGTKLEIKESKYGPPCPPCPAPEAAGGPSVFL FPPKPKDTLMISRTPEVTCVVVDVSQEDPEVQFNWYVDGVEV | SEQ ID NO.25 |
| | HNAKTKPREEQFNSTYRVVSVLTVLHQDWLNGKEYKCKVSN KGLPSSIEKTISKAKGQPREPQVYTLPPSQEEMTKNQVSLTCLV KGFYPSDIAVEWESNGQPENNYKTTPPVLDSDGSFFLYSRLTV DKSRWQEGNVFSCSVMHEALHNHYTQKSLSLSLGK | |
| antiFITC×C D3-Hab01 light chain | DVVMTQTPLSLPVSLGDQASISCRSSQSLVHSNGNTYLRWYL QKPGQSPKVLIYKVSNRFSGVPDRFSGSGSGTDFTLKISRVEAE DLGVYFCSQSTHVPWTFGGGTKLEIKRTVAAPSVFIFPPSDEQL KSGTASVVCLLNNFYPREAKVQWKVDNALQSGNSQESVTEQ DSKDSTYSLSSTLTLSKADYEKHKVYACEVTHQGLSSPVTKSF NRGEC | SEQ ID NO.26 |

**Table 3 Antibody CDR sequences determined by Kabat numbering system**

| **Designation of sequence** | **CDR1** | **CDR2** | **CDR3** |
|---|---|---|---|
| BCMA-VHH01 | YYMIG SEQ ID NO.27 | SISPADGSTYYADSVKG SEQ ID NO.28 | GNEATISWGFGPWEYDY SEQ ID NO.29 |
| BCMA-VHH02 | IHIMA SEQ ID NO.30 | GIRNDGSTVYVDSVKG SEQ ID NO.31 | DQGFGSYSEWERRSR SEQ ID NO.32 |
| BCMA-Hab01 VH | DHTFH SEQ ID NO.33 | YIYPRDQNTKYNEKFKG SEQ ID NO.34 | AGYYGSSHAMDY SEQ ID NO.35 |
| BCMA-Hab01 VL | KASQDVSTAVA SEQ ID NO.36 | SASSRYT SEQ ID NO.37 | QQLYSTPWT SEQ ID NO.38 |
| GPRC5D-Hab01VH | SYGIS SEQ ID NO.39 | EIYPRSGNTYYNEKFKG SEQ ID NO.40 | GRAYGRNYPMDY SEQ ID NO.41 |
| GPRC5D-Hab01VL | RASQSIGTSIH SEQ ID NO.42 | YASESIS SEQ ID NO.43 | QQSNSWPYT SEQ ID NO.44 |
| GPRC5D-Hab02 VH | NFGIT SEQ ID NO.45 | ENYPRRINTYYNEKFKG SEQ ID NO.46 | KDIFGLSYALDY SEQ ID NO.47 |
| GPRC5D-Hab02 VL | KASQNVGTNVA SEQ ID NO.48 | SASYQYS SEQ ID NO.49 | QQYKNYPYT SEQ ID NO.50 |
| CD3-Hab01VH | DYYMS SEQ ID NO.51 | MSRNKAKGHTIEYSSSVKG SEQ ID NO.52 | DTYESYDGYFDV SEQ ID NO.53 |
| CD3-Hab01VL | KSSQSLFNSRSRKNY LA SEQ ID NO.54 | WASIRES SEQ ID NO.55 | KQSYYLYT SEQ ID NO.56 |

### Example 2 Expression and purification of anti-BCMA-GPRC5D-CD3 trispecific antibody molecule and control antibody

### 2.1 Expression and purification of trispecific antibody molecule

A plasmid and a transfection reagent PEI (Polysciences, catalog number: 24765-1) were added to OPTI-MEM (Gibco, catalog number: 11058021), mixed uniformly, and then left to stand for 15 min. Expi293F cells (Thermofisher, catalog number: A14527) were added to the mixture and cultured at 37°C under 5% CO₂ in a shaker at 120 rpm. On the second day of transfection, OPM-293 ProFeed (Shanghai OPM Biosciences Co., Ltd., catalog number: F081918-001) and 6 g/L glucose (Sigma, catalog number: G7528) were added. On the sixth day of transfection, the cell supernatant was collected. After the culture supernatant was collected, Protein A affinity purification and molecular sieve purification were performed on the protein by AKTA Pure. The obtained antibodies were analyzed quantitatively and qualitatively by SDS-PAGE, SEC-HPLC and CE-SDS. According to different structures of the antibodies, purification can be performed by the following methods respectively:

### (A) Method for purifying symmetrical trispecific antibody

The method was suitable for the purification of trispecific antibodies Tri-17, Tri-29, Tri-32, Tri-35, Tri-55, Tri-59 and Tri-60. The specific purification method was as follows:
1. Primary purification was performed with Protein A column (Mabselect SuRe^{™}, purchased from Cytiva). The Protein A column was first balanced with 3-5 times the column volume of an equilibration buffer (a PBS buffer, pH 7.4), and then the clarified culture supernatant was loaded on the column at a flow rate of 8 mL/min. After loading, the column was eluted with 3-5 times the column volume of a high-salt eluent (20 mM phosphate buffer, 1 M NaCl, pH 7.4). The protein bound to Protein A column was eluted with an eluent (20 mM citric acid buffer, pH 3.5), and the elution of the protein was monitored according to the A₂₈₀ ultraviolet absorption peak. The eluted protein was collected, and neutralized to pH 5-6 by adding 1 M pH 8.0 Tris-HCl.
2. A molecular sieve (Ezload 16/60 Chromdex 200 pg purchased from Bestchrom or Superdex 200 Increase 10/300 GL purchased from Cytiva) was used for the fine purification and the target sample was collected. After concentration, the solution was changed to 559 buffer (10 mM acetic acid, 9% sucrose, pH 5.5) by dialysis. Aseptic filtration was performed with a 0.22 µm filter, and the sample was aseptically preserved to obtain the purified corresponding antibody.

### (B) Method for purifying class I asymmetric trispecific antibody

The method was suitable for the purification of trispecific antibodies Tri-38, Tri- 41, Tri-46, Tri-49, Tri- 52, Tri-56 and Tri-62. The specific purification method was as follows:
1. Primary purification was performed with Protein A column. The Protein A column was first balanced with 3-5 times the column volume of an equilibration buffer (a PBS buffer, pH 7.4), and then the clarified culture supernatant was loaded on the column at a flow rate of 8 mL/min. After loading, the column was eluted with 3-5 times the column volume of a high-salt eluent. The redundant half-antibody was removed with eluent A (20 mM citric acid buffer, pH 3.85), the target protein was eluted with eluent B (20 mM citric acid buffer, pH 3.5), and the elution of the protein was monitored according to the A₂₈₀ ultraviolet absorption peak. The eluted protein was collected, and neutralized to pH 5-6 by adding 1 M pH 8.0 Tris-HCl.
2. A molecular sieve was used for the fine purification and the target sample was collected. After concentration, the solution was changed to 559 buffer by dialysis. Aseptic filtration was performed with a 0.22 µm filter, and the sample was aseptically preserved to obtain the purified corresponding antibody.

### (C) Method for purifying class II asymmetric trispecific antibody

The method was suitable for the purification of trispecific antibodies Tri-63, Tri-64, Tri-65 and Tri-66. The specific purification method was as follows:
1. Primary purification was performed with Protein A column. The Protein A column was first balanced with 3-5 times the column volume of an equilibration buffer (a PBS buffer, pH 7.4), and then the clarified culture supernatant was loaded on the column at a flow rate of 8 mL/min. After loading, the column was eluted with 3-5 times the column volume of a high-salt eluent. The protein bound to Protein A column was eluted with an eluent (20 mM citric acid buffer, pH 3.5), and the elution of the protein was monitored according to the A280 ultraviolet absorption peak. The eluted protein was collected, and neutralized to pH 5-6 by adding 1 M pH 8.0 Tris-HCl.
2. Fine purification was performed with HiTrap KappaSelect (purchased from Cytiva). The column was first balanced with 3-5 times the column volume of an equilibration buffer (a PBS buffer, pH 7.4), and then the primarily purified protein was loaded on the column at a flow rate of 5 mL/min. After loading, the column was eluted with 3-5 times the column volume of PBS, followed by 10 times the column volume of a 10 mM phosphate buffer (pH 7.4). The protein bound to KappaSelect column was eluted with eluent C (50 mM glycine, pH 3.6), the impurity protein was eluted with eluent D (50 mM glycine, pH 3.0), and the elution of the protein was monitored according to the A280 ultraviolet absorption peak. The eluted protein was collected, and neutralized to pH 5-6 by adding 1 M pH 8.0 Tris-HCl.
3. A molecular sieve was used for the fine purification and the target sample was collected. After concentration, the solution was changed to 559 buffer by dialysis. Aseptic filtration was performed with a 0.22 µm filter, and the sample was aseptically preserved to obtain the purified corresponding antibody.

For the trispecific antibodies purified by different methods, protein samples to be detected were analyzed by SEC-HPLC to characterize the molecular size homogeneity and purity of the proteins. The HPLC used was Agilent 1260, the chromatographic column TSKgel G3000SWXL was from Tosoh Bioscience, the mobile phase was 200 mM phosphate buffer (pH 7.0)/isopropanol (v/v 9 : 1), the detection temperature was 25°C, the flow rate was 0.5 mL/min, and the detection wavelength was 280 nm. For the SEC-HPLC data, the chromatogram was analyzed by a manual integration method, and the protein purity was calculated by an area normalization method. The main peak was considered as a monomer, the chromatographic peak before the main peak was called an aggregate, and the chromatographic peak after the main peak was called a fragment. The results were as shown in Table 4.

**Table 4: Trispecific antibody molecule expression quantity and SEC purity results**

| **Designation of antibody** | **Expression system** | **Expression quantity per unit volume (mg/L)** | **SEC monomer purity (%)** |
|---|---|---|---|
| Tri-17 | Expi293F | 14.54 | 92.48 |
| Tri-29 | Expi293F | 42.66 | 97.61 |
| Tri-32 | Expi293F | 69.80 | 91.81 |
| Tri-35 | Expi293F | 47.36 | 94.43 |
| Tri-38 | Expi293F | 38.44 | 97.77 |
| Tri-41 | Expi293F | 43.74 | 97.76 |
| Tri-46 | Expi293F | 28.54 | 97.73 |
| Tri-49 | Expi293F | 7.16 | 96.72 |
| Tri-52 | Expi293F | 25.15 | 94.68 |
| Tri-55 | Expi293F | 31.78 | 91.55 |
| Tri-56 | Expi293F | 8.14 | 95.35 |
| Tri-59 | Expi293F | 8.37 | 97.00 |
| Tri-60 | Expi293F | 13.86 | 97.88 |
| Tri-62 | Expi293F | 10.23 | 96.87 |
| Tri-63 | Expi293F | 6.21 | 98.45 |
| Tri-64 | Expi293F | 7.59 | 97.41 |
| Tri-65 | Expi293F | 4.99 | 97.31 |
| Tri-66 | Expi293F | 8.47 | 97.49 |

### 2.2 Expression and purification of control antibody

Anti-hel-hIgG1 (commonly abbreviated as hIgG1 in the present application) was an antibody targeting chicken egg lysozyme (purchased from Biointron, catalog number: B117901), which is a common negative control antibody used in the process of screening and identifying an antibody. The method for expressing REGN5459, JNJ7564 and antiFITC×CD3-Hab01 was the same as the expression of trispecific antibody molecule in example 2.1. The purification methods were as follows:
(A) Method for purifying REGN5459 and antiFITC×CD3-Hab01
   1. Primary purification was performed with Protein A column. The Protein A column was first balanced with 3-5 times the column volume of an equilibration buffer (a PBS buffer, pH 7.4), and then the clarified culture supernatant was loaded on the column at a flow rate of 8 mL/min. After loading, the column was eluted with 3-5 times the column volume of a high-salt eluent (20 mM phosphate buffer, 1 M NaCl, pH 7.4). The target protein was eluted with eluent A (20 mM citric acid buffer, pH 3.85) and eluent B (20 mM citric acid buffer, pH 3.5) at a linear gradient, and the elution of the protein was monitored by the A280 ultraviolet absorption peak. The eluted protein was collected, and neutralized to pH 5-6 by adding 1 M pH 8.0 Tris-HCl.
   2. A molecular sieve was used for the fine purification and the target sample was collected, and the sample was finally stored in a PBS buffer. Aseptic filtration was performed with a 0.22 µm filter, and the sample was aseptically preserved to obtain the purified REGN5459 and antiFITC×CD3-Hab01 antibodies.
(B) Method for purifying JNJ7564
   1. The corresponding monospecific antibodies JNJ7564-anti-GPRC5D and JNJ7564- anti-CD3 were respectively purified by Protein A column. The Protein A column was first balanced with 3-5 times the column volume of an equilibration buffer (a PBS buffer, pH 7.4), and then the clarified culture supernatant was loaded on the column at a flow rate of 8 mL/min. After loading, the column was eluted with 3-5 times the column volume of a high-salt eluent (20 mM phosphate buffer, 1 M NaCl, pH 7.4). The protein bound to Protein A column was eluted with an eluent (20 mM citric acid buffer, pH 3.5), and the elution of the protein was monitored according to the A280 ultraviolet absorption peak. The eluted protein was collected, and neutralized to pH 5-6 by adding 1 M pH 8.0 Tris-HCl. The buffer was changed to a PBS buffer by dialysis.
   2. A bispecific antibody was produced by reduction and reoxidation in vitro. 1-20 mg/ml anti-GPRC5D/anti-CD3 antibody were mixed in a molar ratio of 1.08 : 1, 2-mercaptoethanol (2-MEA) with a final concentration of 75 mM was added, and the mixture was incubated at 25-37°C for 2-6 h. The buffer was changed by dialysis or ultrafiltration or desalination to remove 2-mercaptoethanol (2-MEA). The bispecific antibody was produced by oxidation and reassembly at 20°C.

For the trispecific antibodies purified by different methods, protein samples to be detected were analyzed by SEC-HPLC to characterize the molecular size homogeneity and purity of the proteins. The method was the same as above. The results were as shown in Table 5.

**Table 5: Control molecule expression quantity and SEC purity results**

| Designation of antibody | Expression system | Expression quantity per unit volume (mg/L) | SEC monomer purity (%) |
|---|---|---|---|
| REGN5459 | Expi293F | 54.11 | 98.64 |
| JNJ7564 | Expi293F | 14.97 | 97.19 |
| antiFITC×CD3-Hab01 | Expi293F | 49.14 | 94.65 |

### Example 3 Construction and identification of engineering cell line

### 3.1 Preparation of CHOK1-human GPRC5D cells and CHOK1-monkey GPRC5D cells

Nucleotide sequences encoding human GPRC5D amino acid sequence (NCBI Gene ID: 55507) and monkey GPRC5D amino acid sequence (NCBI Gene ID: 102122120) were cloned into a pLVX lentiviral vector respectively, and viral particles were prepared in HEK293T cells. CHOK1 cell line was infected with lentiviruses, and then selectively cultured in Advanced DMEM/F12 Medium (purchased from Gibco, catalog number: 12634028) containing 10 µg/ml puromycin (purchased from Gibco, catalog number A1113803) and 10% (w/w) fetal bovine serum (purchased from ExCell Bio, catalog number: FND500) for 1 week. JNJ7564 was used as a primary antibody and goat anti-human IgG (H+L) antibody (Jackson, catalog number: 109605088) was used as a secondary antibody. The detection was performed by a flow cytometer FACS CantoII (purchased from BD Biosciences). The cells having a high expression quantity and a single peak shape were amplified, and the amplified cells were re-detected by a flow cytometry method. The positive cell pool with better growth, higher fluorescence intensity and better homogeneity was selected for further scale-up culture and then cryopreserved in liquid nitrogen.

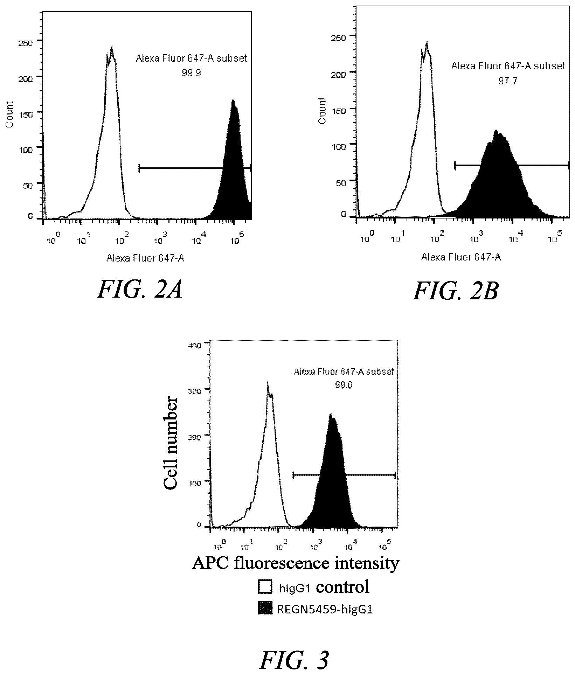

The previously prepared JNJ7564-anti-GPRC5D-hIgG1 (GPRC5D-VH and VL sequences from JNJ7564 were cloned into expression vectors PTT5-huIgG1 and PTT5-huIgGLC(κ) comprising a signal peptide MGWSWILLFLLSVTAGVHS (SEQ ID NO.57) and a human heavy/light chain constant region, respectively, to construct a chimeric monoclonal antibody, which was commissioned to General Biosystems (Anhui) Co., Ltd.) was used for identification, and the antibody had a cross reactivity to human and monkey GPRC5D. The FACS results were as shown in FIGs. 2A-2B, showing a single peak, which can be used to monitor the binding activity of the multi-specific binding molecules to human and monkey GPRC5D.

### 3.2 Preparation of cell line Flp-inCHO-human BCMA cells overexpressing target antigen BCMA

A nucleotide sequence encoding human BCMA amino acid sequence (NCBI Gene ID: 608) was cloned into a PcDNA5/FRT vector (purchased from Clontech). Flp-in CHO cell line was transfected, and then selectively cultured in F12K Medium (purchased from Gibco, catalog number: 21127030) containing 600 µg/ml hygromycin (purchased from ThermoFisher, catalog number 10687010) and 10% (w/w) fetal bovine serum (purchased from ExCell Bio, catalog number: FND500) for 2 weeks. REGN5459 antibody was used as a primary antibody and goat anti-human IgG (H+L) antibody (Jackson, catalog number: 109605088) was used as a secondary antibody. The detection was performed by a flow cytometer FACS CantoII (purchased from BD Biosciences). The cells having a high expression quantity and a single peak shape were amplified, and the amplified cells were re-detected by a flow cytometry method. The positive cell pool with better growth, higher fluorescence intensity and better homogeneity was selected for further scale-up culture and then cryopreserved in liquid nitrogen.

The previously prepared REGN5459-anti-BCMA-hIgG1 (BCMA-VH and VL sequences from REGN5459 were cloned into expression vectors PTT5-huIgG1 and PTT5-huIgGLC(κ) comprising a signal peptide MGWSWILLFLLSVTAGVHS and a human heavy/light chain constant region, respectively, to construct a chimeric monoclonal antibody, which was commissioned to General Biosystems (Anhui) Co., Ltd.) was used for identification, and the antibody had a cross reactivity to human and monkey BCMA. The FACS results were as shown in FIG. 3, showing a single peak, which can be used to monitor the binding activity of the multi-specific binding molecules to human BCMA.

### 3.3 Preparation of luciferase reporter gene stable transformation cell line

A pLvx-luciferase-P2A-GFP-RES-hygro vector system was subjected to lentivirus packaging, and then Molp-8 cells were virally infected. After 24 h, the buffer was changed and 0.125 mg/ml hygro was added for pressure screening; and after pressure screening with hygro for 2 weeks, the GFP expression kurtosis detected by FACS was about 40 times, and cell pool was successfully infected. The cells were plated using a limited dilution method at 1 cell/well onto a total of 3 pieces of 96-well cell plates, and cultured at 37°C for 2-3 weeks, and a total of about 30 monoclonal cell lines grew; and these monoclonal cells were transferred to the cells of 24-well plates, and then cultured for 3-4 days, and according to cell growth status and GFP intensity, 3 monoclonal cell lines were selected for scale-up culture, the expression of luciferase in the cells was further detected, and finally, the monoclonal Molp-8-Luc cells with strong fluorescence were obtained. H929-Luc pool and RPMI8226-Luc pool were both purchased from Nanjing Cobioer Biosciences Co., Ltd., and the monoclonal cells were prepared by the same method as that for Molp-8-Luc.

### Example 4 Identification of binding of anti-BCMA-GPRC5D-CD3 trispecific antibody molecule to target antigen and CD3

### (A) Detection of binding of anti-BCMA-GPRC5D-CD3 trispecific antibody to human BCMA protein and cross-binding of anti-BCMA-GPRC5D-CD3 trispecific antibody to monkey BCMA protein by enzyme-linked immunosorbent assay (ELISA)

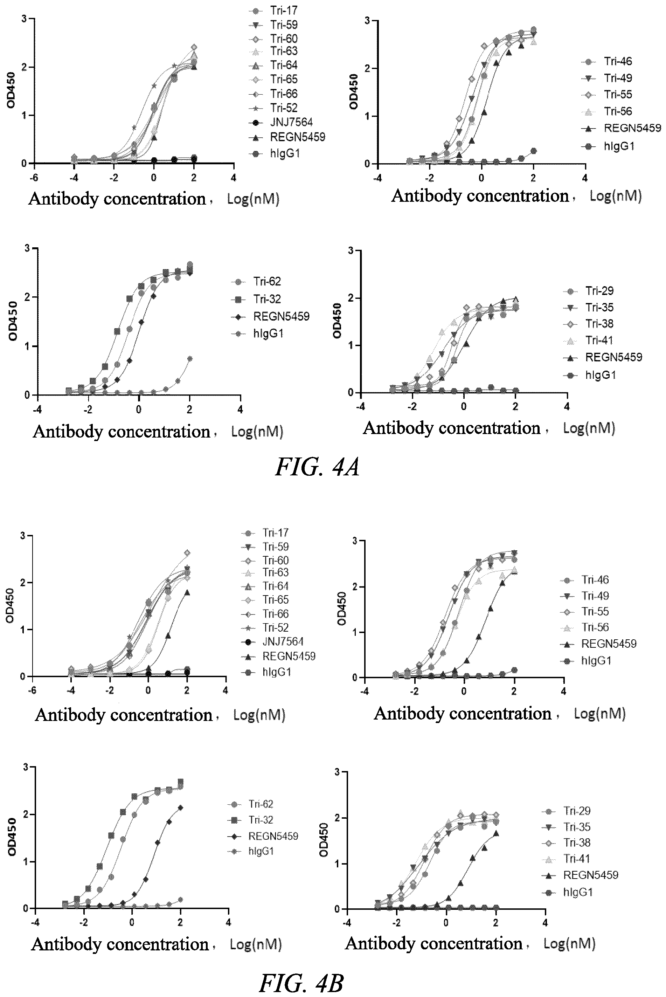

The specific method was as follows: Human BCMA protein (purchased from Acro, catalog number: BCA-H522y) and monkey BCMA protein (purchased from Acro, catalog number: BCA-C52H7) were diluted with PBS to a final concentration of 1 µg/mL, respectively, and then added to a 96-well ELISA plate at 50 µl/well. The plate was sealed with a plastic film and incubated overnight at 4°C, the plate was washed twice with PBS the next day, and then a blocking solution [PBS+2% (w/w) BSA] was added for blocking at room temperature for 2 h. The blocking solution was discarded, the plate was washed 3 times with PBS, and 100 nM anti-BCMA-GPRC5D-CD3 antibody diluted gradiently or control antibody was added at 50 µl/well. After incubation at 37°C for 2 h, the plate was washed 3 times with PBS. HRP (horseradish peroxidase)-labeled goat anti-human secondary antibody (purchased from Merck, catalog number: AP113P) was added, and incubated at 37°C for 1 h, and the plate was washed 5 times with PBS. TMB substrate was added at 50 µl/well, and incubated at room temperature for 10 min, then a stop solution (1.0 M HCl) was added at 50 µl/well. The OD450nm values were read by an ELISA microplate reader (Multimode Plate Reader, EnSight, purchased from Perkin Elmer). The results were as shown in FIGs. 4A and 4B, indicating that all the trispecific antibody molecules can bind to human BCMA and have a cross-binding activity against monkey BCMA. IgG subtype control was human IgG1 (anti-HEL-IgG1).

### (B) Detection of binding of anti-BCMA-GPRCSD-CD3 trispecific antibody to CHOK1-human GPRC5D cells and binding of anti-BCMA-GPRC5D-CD3 trispecific antibody to CHOK1-monkey GPRC5D by cell-based ELISA

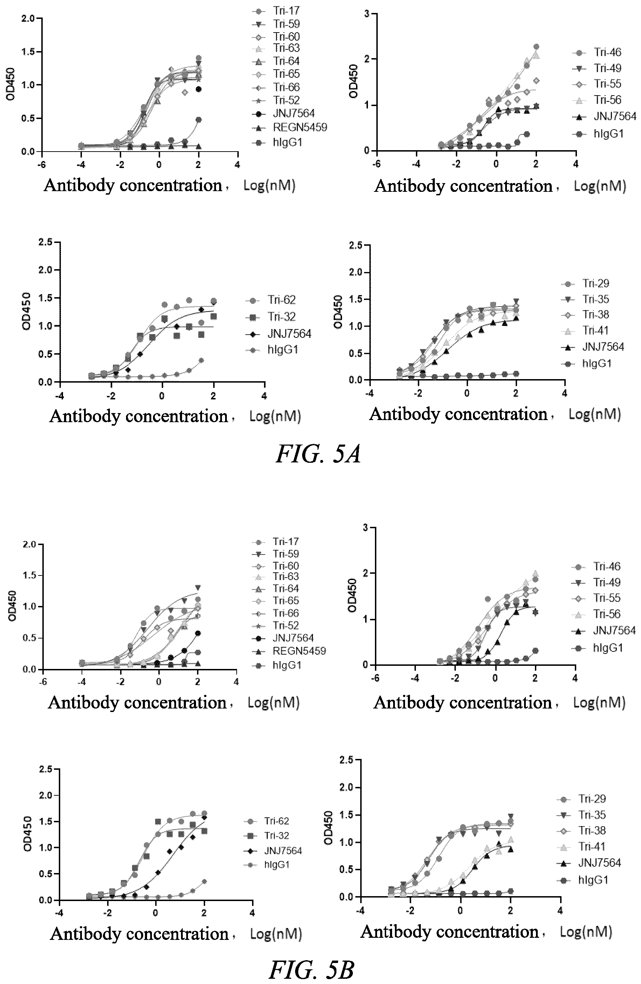

The specific method was as follows: The required cells were scale-up cultured in a T-75 cell culture flask to the logarithmic growth phase, the medium was aspirated, the cells were washed twice with a PBS buffer, and then digested with trypsin. Then the digestion was terminated with a complete medium, and the cells were pipetted to obtain a single cell suspension. After the cells were counted and centrifuged, the cell precipitate was resuspended to 4 × 10⁵ cells per milliliter with a complete medium, and the suspension was added to a 96-well flat-bottomed cell culture plate at 100 µl/well, and cultured overnight in a 37°C, 5% carbon dioxide incubator. On the next day, the medium in the 96-well plate was discarded, the cells were washed once with PBS, and then fixed at room temperature for 0.5 h with an immunostaining fixative (purchased from Beyotime, catalog number: P0098-500ml) at 50 µl/well. A blocking solution [PBS+5% (w/v) skim milk] was added for blocking at room temperature for 2 h. The blocking solution was discarded, the plate was washed 3 times with PBS, and 100 nM anti-BCMA-GPRC5D-CD3 antibody diluted gradiently or control antibody was added at 50 µl/well. After incubation at 37°C for 2 h, the plate was washed 3 times with PBS. HRP (horseradish peroxidase)-labeled goat anti-human secondary antibody was added, and incubated at 37°C for 1 h, and the plate was washed 5 times with PBS. TMB substrate was added at 50 µl/well, and incubated at room temperature for 10 min, then a stop solution (1.0 M HCl) was added at 50 µl/well. The OD450nm values were read by an ELISA microplate reader. The results were as shown in FIGs. 5A and 5B, indicating that all the trispecific antibody molecules can bind to CHOK1-human GPRC5D cells and have a cross-binding activity against CHOK1-monkey GPRC5D cells.

### (C) Detection of binding of anti-BCMA-GPRC5D-CD3 trispecific antibody to human CD3e/d protein and binding of anti-BCMA-GPRC5D-CD3 trispecific antibody to human CD3e protein by enzyme-linked immunosorbent assay (ELISA)

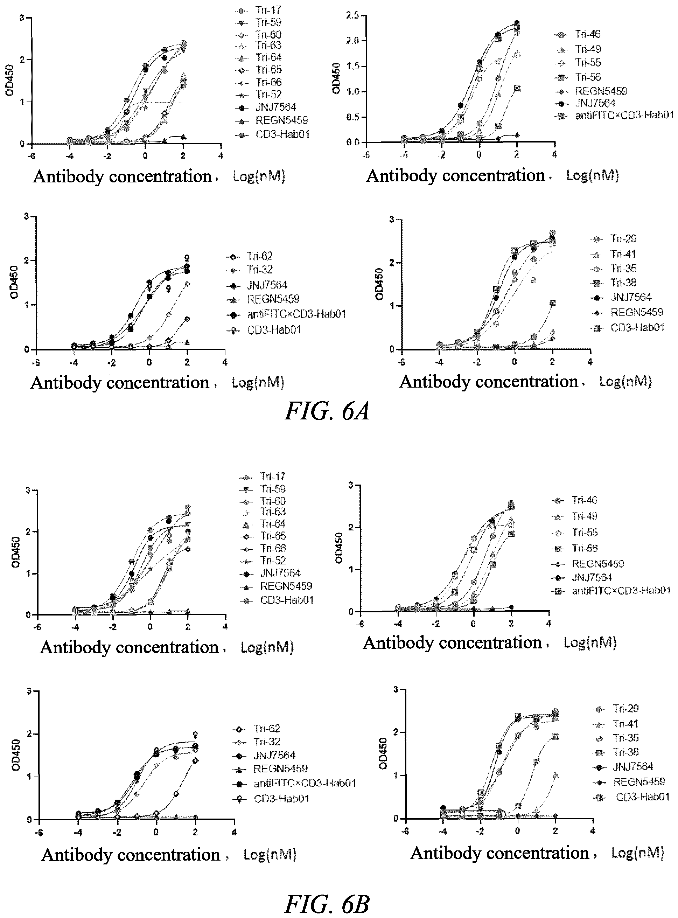

The specific method was the same as that of example 4 (A), wherein proteins for coating a 96-well ELISA plate were human CD3e/d protein (purchased from Sino Biological, catalog number: CT032-H2508) and human CD3e protein (purchased from Sino Biological, catalog number: 10977-H08H), respectively. The results were as shown in FIGs. 6A and 6B, indicating that the trispecific antibody molecules bind to human CD3e/d protein and human CD3e protein to a certain extent.

### (D) Detection of binding of anti-BCMA-GPRC5D-CD3 trispecific antibody to cell line overexpressing target antigen BCMA and binding of anti-BCMA-GPRC5D-CD3 trispecific antibody to cell line overexpressing target antigen GPRC5D by fluorescence activated cell sorting (FACS)

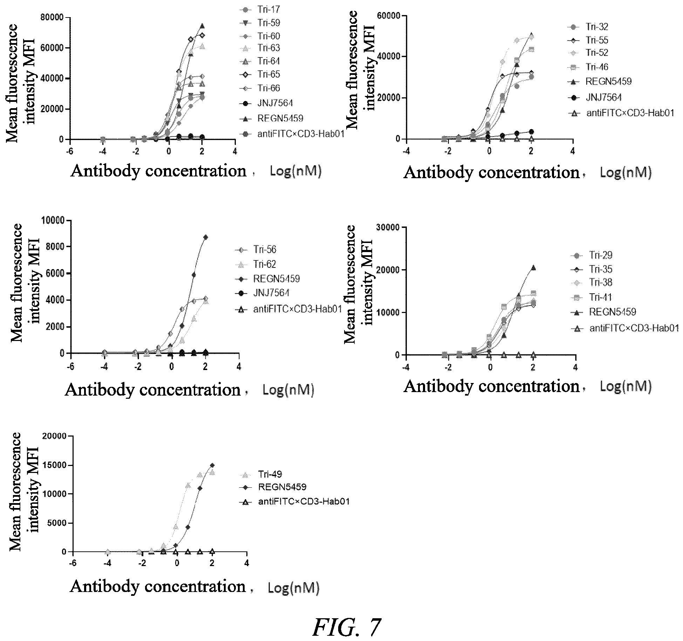

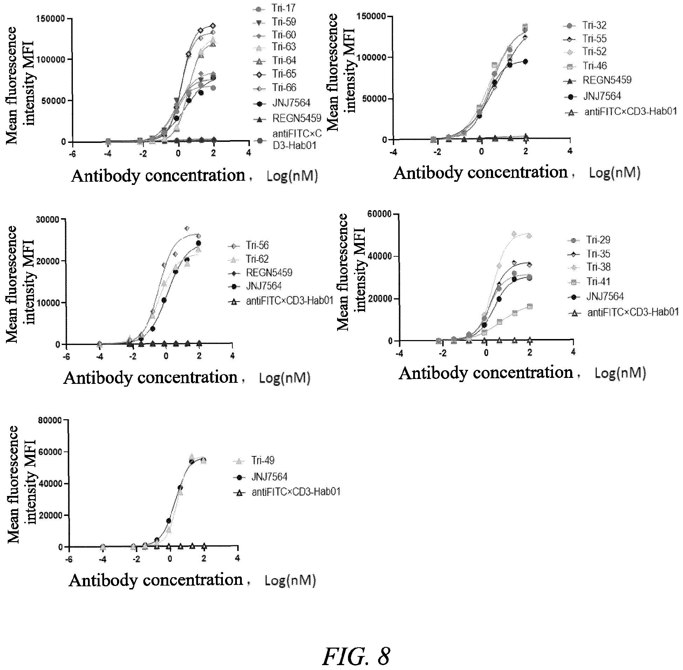

Flp-inCHO-human BCMA cells and CHOK1-human GPRC5D cells were scale-up cultured in T-75 cell culture flask to the logarithmic growth phase, respectively, and the cells were pipetted to obtain a single cell suspension. After the cells were counted and centrifuged, the cell precipitate was resuspended to 2 × 10⁶ cells per milliliter with a FACS buffer (PBS+2% fetal bovine serum). The suspension was added to a 96-well FACS reaction plate at 50 µl/well. 8 concentrations of the anti-BCMA-GPRC5D-CD3 antibodies or control antibodies were added at 50 µl/well, wherein gradient dilution was performed at a dilution ratio of 5 from a concentration of 100 nM. The cells were incubated at 4°C for 1 h. The obtained mixture was centrifuged and washed 3 times with a PBS buffer. Secondary antibody anti-hIgG(Fc) Alexa 647 (purchased from Jackson, catalog number: 109-605-098) was added at 50 µl/well, and incubated on ice for 1 h. The obtained mixture was centrifuged and washed 3 times with a PBS buffer. 100 µl of the mixture was taken for detection and result analysis by FACS (FACS CantoTM, purchased from BD Company). Data analysis was performed by software (FlowJo) to obtain the mean fluorescence intensity (MFI) of the cells. Then, a software (GraphPad Prism8) was used for analysis, data fitting and EC50 calculation. The results were as shown in Table 6, FIG. 7, Table 7 and FIG. 8, indicating that all the trispecific antibody molecules bind well to target antigens BCMA and GPRC5D overexpressed on cells, respectively.

**Table 6: Detection of binding activity of trispecific antibody against Flp-inCHO-human BCMA cells by FACS**

| **Designation of antibody** | **Flp-inCHO-human BCMA** | **Designation of antibody** | **Flp-inCHO-human BCMA** |
|---|---|---|---|
| | **EC50 (nM)** | | **EC50 (nM)** |
| Tri-17 | 2.97 | Tri-56 | 1.30 |
| Tri-59 | 1.21 | Tri-62 | 14.53 |
| Tri-60 | 6.84 | REGN5459 | 13.87 |
| Tri-63 | 2.10 | JNJ7564 | Negative |
| Tri-64 | 1.08 | antiFITC×CD3-Hab01 | Negative |
| Tri-65 | 2.54 | Tri-29 | 2.27 |
| Tri-66 | 1.09 | Tri-35 | 2.53 |
| REGN5459 | 9.37 | Tri-38 | 4.43 |
| JNJ7564 | Negative | Tri-41 | 1.52 |
| antiFITC×CD3-Hab01 | Negative | REGN5459 | 13.28 |
| Tri-32 | 2.03 | antiFITC×CD3-Hab01 | Negative |
| Tri-55 | 0.92 | Tri-49 | 1.34 |
| Tri-52 | 1.88 | REGN5459 | 10.40 |
| Tri-46 | 5.19 | antiFITC×CD3-Hab01 | Negative |
| REGN5459 | 11.33 | / | |
| JNJ7564 | Negative | | |
| antiFITC×CD3-Hab01 | Negative | | |

**Table 7: Detection of binding activity of trispecific antibody against CHOK1-human GPRC5D cells by FACS**

| **Designation of antibody** | **CHOK1-human GPRC5D** | **Designation of antibody** | **CHOK1-human GPRC5D** |
|---|---|---|---|
| | **EC50 (nM)** | | **EC50 (nM)** |
| Tri-17 | 0.69 | Tri-56 | 0.38 |
| Tri-59 | 0.59 | Tri-62 | 0.40 |
| Tri-60 | 0.92 | JNJ7564 | 1.28 |
| Tri-63 | 3.80 | REGN5459 | Negative |
| Tri-64 | 3.63 | antiFITC×CD3-Hab01 | Negative |
| Tri-65 | 1.66 | Tri-29 | 1.39 |
| Tri-66 | 1.53 | Tri-35 | 1.78 |
| JNJ7564 | 2.32 | Tri-38 | 2.06 |
| REGN5459 | Negative | Tri-41 | 4.30 |
| antiFITC×CD3-Hab01 | Negative | JNJ7564 | 2.27 |
| Tri-32 | 3.06 | antiFITC×CD3-Hab01 | Negative |
| Tri-55 | 6.26 | Tri-49 | 2.75 |
| Tri-52 | 2.97 | JNJ7564 | 2.00 |
| Tri-46 | 2.36 | antiFITC×CD3-Hab01 | Negative |
| JNJ7564 | 1.73 | / | |
| REGN5459 | Negative | | |
| antiFITC×CD3-Hab01 | Negative | | |

### (E) Detection of binding of anti-BCMA-GPRCSD-CD3 trispecific antibody to endogenous tumor cell co-expressing target antigens BCMA and GPRC5D by FACS

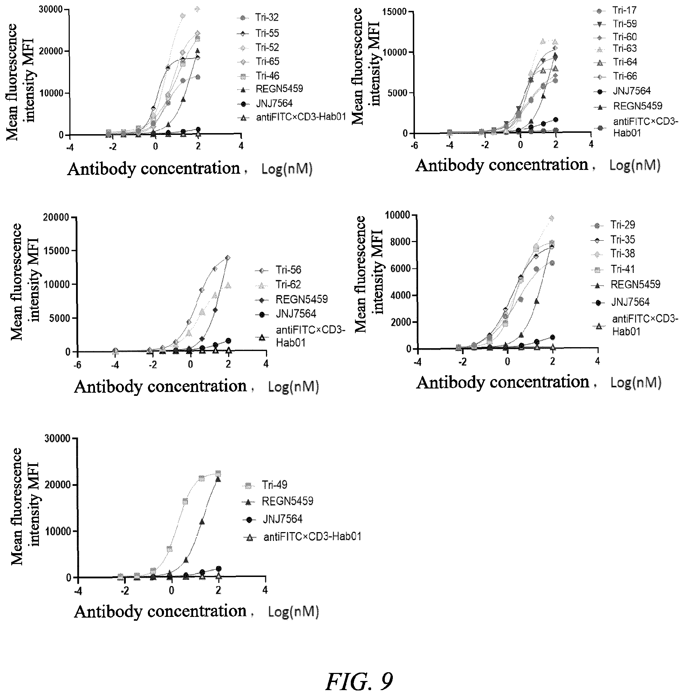

The specific method was the same as that of example 4 (D). In this experiment, a multiple myeloma cell line NCI-H929 (purchased from ATCC) was selected as an endogenous cell to detect the binding ability of the trispecific antibody molecules, and NCI-H929 was a cell line with a high expression of both GPRC5D and BCMA. The results were as shown in Table 8 and FIG. 9, indicating that the trispecific antibody molecules can strongly bind to an endogenous tumor cell NCI-H929 expressing antigens BCMA and GPRC5D.

**Table 8: Detection of binding activity of trispecific antibodies against H929 cells by FACS**

| **Designation of antibody** | **H929** | | **Designation of antibody** | **H929** | |
|---|---|---|---|---|---|
| | **Maximum mean fluorescence intensity** | **EC50 (nM)** | | **Maximum mean fluorescence intensity** | **EC50 (nM)** |
| Tri-32 | 13535 | 3.53 | Tri-56 | 13799 | 2.20 |
| Tri-55 | 18187 | 1.23 | Tri-62 | 9728 | 3.13 |
| Tri-52 | 29813 | 3.65 | REGN5459 | 13772 | 55.09 |
| Tri-46 | 22659 | 10.27 | JNJ7564 | 1485 | 34.74 |
| Tri-65 | 23977 | 6.32 | antiFITC×CD3-Hab01 | 118 | Negative |
| REGN5459 | 19901 | 61.73 | Tri-29 | 6347 | 2.67 |
| JNJ7564 | 1052 | 30.24 | Tri-35 | 7546 | 1.55 |
| antiFITC ×CD3-Hab01 | 95 | Negative | Tri-38 | 9714 | 5.68 |
| Tri-17 | 6399 | 2.53 | Tri-41 | 7802 | 2.38 |
| Tri-59 | 9069 | 1.82 | REGN5459 | 7875 | 50.50 |
| Tri-60 | 7034 | 3.15 | JNJ7564 | 807 | 36.35 |
| Tri-63 | 11266 | 2.70 | antiFITC×CD3-Hab01 | 166 | Negative |
| Tri-64 | 7893 | 1.32 | Tri-49 | 22245 | 1.82 |
| Tri-66 | 10410 | 2.83 | REGN5459 | 21083 | 23.97 |
| JNJ7564 | 1591 | 20.62 | JNJ7564 | 1706 | 32.71 |
| REGN5459 | 9578 | 44.74 | antiFITC×CD3-Hab01 | 210 | Negative |
| antiFITC×C D3-Hab01 | 294 | Negative | / | | |

### (F) Detection of binding of anti-BCMA-GPRC5D-CD3 trispecific antibody to endogenous tumor cell expressing CD3 and binding of the anti-BCMA-GPRC5D-CD3 trispecific antibody to activated human PBMC and monkey PBMC cells expressing CD3 by FACS

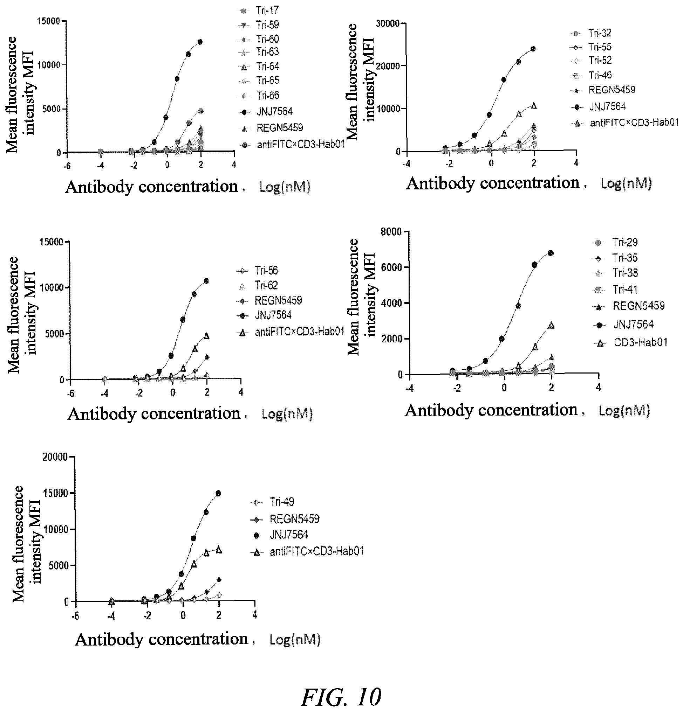

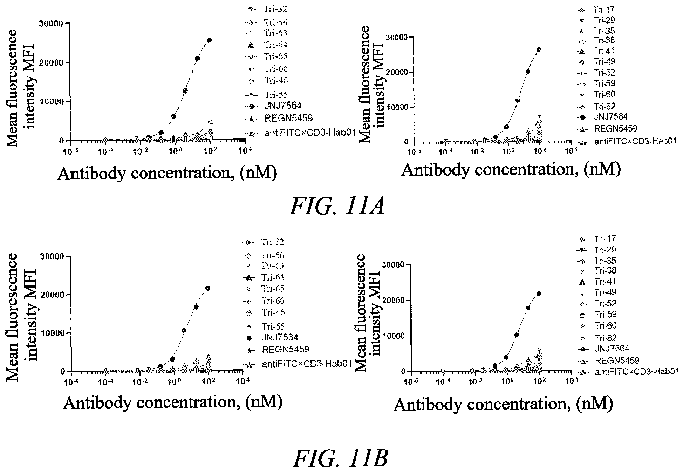

The endogenous cell expressing CD3 was Jurkat (purchased from ATCC). The process of preparing human PBMC and monkey PBMC cells expressing CD3 was as follows: PBMC isolated from human blood was activated and cultured for 3 days according to the methods of a human T cell activation kit (purchased from Miltenyi Biotec, catalog number: 130-091-441) and a monkey T cell activation kit (purchased from Miltenyi Biotec, catalog number: 130-092-919), respectively, and then the activated T cells were cultured with a 1640 medium containing 100 units of human IL2 protein (purchased from Acro, catalog number: IL2-H4113) and 10% (v/v) fetal bovine serum for 2 weeks. The detection method by FACS was the same as that of example 4 (D). The results were as shown in Table 9, FIG. 10, Table 10, FIG. 11A and FIG. 11B, indicating that the trispecific antibody molecules bind to Jurkat cells, human PBMC cells and monkey PBMC cells to a certain extent.

**Table 9. Detection of binding activity of trispecific antibodies against Jurkat cells by FACS**

| **Designation of antibody** | **Jurkat** | | **Designation of antibody** | **Jurkat** | |
|---|---|---|---|---|---|
| | **Maximum mean fluorescence intensity** | **EC50 (nM)** | | **Maximum mean fluorescence intensity** | **EC50 (nM)** |
| Tri-17 | 1152 | The fit is poor | Tri-56 | 356 | The fit is poor |
| Tri-59 | 1804 | The fit is poor | Tri-62 | 462 | The fit is poor |
| Tri-60 | 2233 | The fit is poor | REGN5459 | 2333 | 74.74 |
| Tri-63 | 989 | The fit is poor | JNJ7564 | 10578 | 2.90 |
| Tri-64 | 478 | The fit is poor | antiFITC×CD3-Hab01 | 4667 | 12.01 |
| Tri-65 | 911 | The fit is poor | Tri-29 | 418 | The fit is poor |
| Tri-66 | 550 | The fit is poor | Tri-35 | 320 | The fit is poor |
| JNJ7564 | 12481 | 2.04 | Tri-38 | 204 | The fit is poor |
| REGN5459 | 2692 | The fit is poor | Tri-41 | 85 | Negative |
| antiFITC×CD3-Hab01 | 4612 | 19.14 | JNJ7564 | 6738 | 3.27 |
| Tri-32 | 3123 | 369.7 | REGN5459 | 912 | 49.69 |
| Tri-55 | 4690 | 178.7 | CD3-Hab01 | 2724 | 23.79 |
| Tri-52 | 1054 | The fit is poor | Tri-49 | 795 | 157.4 |
| Tri-46 | 1528 | The fit is poor | JNJ7564 | 14803 | 3.51 |
| REGN5459 | 5867 | 66.79 | REGN5459 | 2918 | 101.1 |
| JNJ7564 | 23795 | 1.82 | antiFITC×CD3-Hab01 | 7073 | 1.79 |
| antiFITC×CD3-Hab01 | 10474 | 6.77 | / | | |

**Table 10. Detection of binding activity of trispecific antibodies against human PBMC and monkey PBMC cells by FACS**

| **Designation of antibody** | **Human PBMC** | | **Monkey PBMC** | |
|---|---|---|---|---|
| | **Maximum mean fluorescence intensity** | **EC50 (nM)** | **Maximum mean fluorescence intensity** | **EC50 (nM)** |
| Tri-32 | 1777 | The fit is poor | 1682 | The fit is poor |
| Tri-56 | 359 | The fit is poor | 575 | The fit is poor |
| Tri-63 | 1340 | The fit is poor | 434 | The fit is poor |
| Tri-64 | 954 | The fit is poor | 348 | The fit is poor |
| Tri-65 | 418 | The fit is poor | 377 | The fit is poor |
| Tri-66 | 313 | The fit is poor | 345 | The fit is poor |
| Tri-46 | 1223 | The fit is poor | 416 | The fit is poor |
| Tri-55 | 2264 | 149.1 | 2032 | 158.9 |
| JNJ7564 | 25390 | 5.21 | 21427 | 5.79 |
| REGN5459 | 2116 | 223.2 | 922 | The fit is poor |
| antiFITC×CD3-Hab01 | 4605 | The fit is poor | 3450 | 15.25 |
| Tri-17 | 2168 | The fit is poor | 3279 | The fit is poor |
| Tri-29 | 6729 | 160.9 | 5634 | The fit is poor |
| Tri-35 | 2236 | 81.36 | 1968 | The fit is poor |
| Tri-38 | 1100 | The fit is poor | 1444 | The fit is poor |
| Tri-41 | 235 | The fit is poor | 417 | The fit is poor |
| Tri-49 | 1730 | 31.79 | 1384 | 59.41 |
| Tri-52 | 1290 | The fit is poor | 693 | The fit is poor |
| Tri-59 | 3514 | 92.06 | 5279 | 249.3 |
| Tri-60 | 2686 | The fit is poor | 3572 | The fit is poor |
| Tri-62 | 452 | The fit is poor | 740 | 85.49 |
| JNJ7564 | 26284 | 7.36 | 21615 | 5.72 |
| REGN5459 | 4366 | 220.9 | 1798 | 161 |
| antiFITC×CD3-Hab01 | 5966 | The fit is poor | 4471 | 12.45 |

### (G) Detection of activation activity of anti-BCMA-GPRC5D-CD3 trispecific antibody to Jurkat-NFAT-NanoLuc cells

Jurkat-NFAT-NanoLuc cells were prepared: Jurkat cells (purchased from ATCC) were seeded in a 6-well culture dish (Costar: 3516) containing 2 mL of a complete medium (RPM1640, 10*%* superior grade FBS, penicillin-streptomycin) at 1E6 cells/well, and continuously cultured at 37°C and 5*%*CO₂ for 24 h. On the next day, 3 µg of pNL[NLucP/NFAT-RE/Hygro] vector and 3 µl of PLUS reagent (Invitrogen: 11514-015) were diluted in 150 µl of Opti-MEM (Gibco: 11058021), and incubated at room temperature for 5 min. The pNL[NLucP/NFAT-RE/Hygro] vector contained NFAT, which drove luciferase reporter gene NanoLuc to transcribe in response to TCR activation. After incubation, the diluted DNA solution was preincubated with a 1 : 1 Lipofectamine LTX solution (Invitrogen: 15338-100) (6 µl of Lipofectamine LTX+144 µl of Opti-MEN), and incubated at room temperature for 15 min to form a DNA-Lipofectamine LTX complex. After incubation, 300 µl of the DNA-Lipofectamine complex was added directly to the cell-containing wells. Jurkat cells were transfected at 37°C and 5*%*CO₂ for 24 h. After incubation, the cells were washed with 3mL of PBS, and selectively cultured for 2 weeks in 1640 (purchased from Gibco, catalog number: 31870082) containing 125 µg/ml hygromycin (Invitrogen: 10687010) and 10*%* (w/w) fetal bovine serum to obtain the required Jurkat-NFAT-NanoLuc cell line.

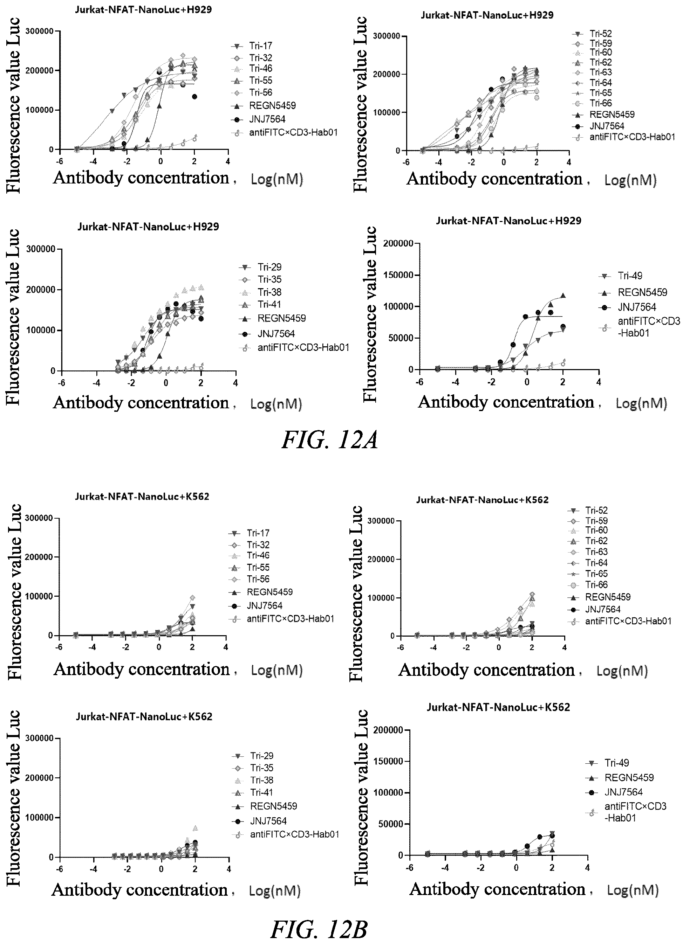

The anti-BCMA-GPRC5D-CD3 trispecific antibody and control antibody were gradiently diluted at a dilution ratio of 5 from 100 nM (final concentration) or gradiently diluted at a dilution ratio of 3 from 100 nM (final concentration), respectively, the diluent being a RPMI 1640 medium containing 5*%* fetal bovine serum. The diluted antibody was added to a 96-well black flat-bottomed light-transmitting cell culture plate at 50 µl/well. H929 cells or negative cells K562 and Jurkat-NFAT-NanoLuc cells were scale-up cultured in a T-75 cell culture flask to the logarithmic growth phase, respectively, and the cells were pipetted to obtain a single cell suspension. After the cells were counted and centrifuged, the cell precipitate was resuspended to 2 × 10⁶ cells per milliliter with a 1640 medium containing 5*%* fetal bovine serum, and the three cells were added to the above 96-well cell culture plate at 25 µl/well, respectively. After uniformly mixing, the cells were incubated in a 37°C, 5*%*(v/v)CO₂ incubator for 5-6 h. After incubation, 50 µl of Nano-Glo luciferase reporter gene detection system (purchased from Promega, catalog number: N1110) was added to each well, and the plate was placed on an oscillator for oscillating incubation for 10 min. Finally, the fluorescence values were read by a plate reader (Multimode Plate Reader, EnSight, purchased from Perkin Elmer). The results were as shown in Table 11 and FIGs. 12A and 12B, indicating that the trispecific antibody molecules can well activate Jurkat-NFAT-NanoLuc cells under the mediation of H929 cells expressing target antigens BCMA and GPRC5D, and do not activate Jurkat-NFAT-NanoLuc cells or produce an extremely weak nonspecific activation under the mediation of negative cells K562 not expressing BCMA and GPRC5D.

**Table 11. Detection of activation activity of trispecific antibodies to Jurkat-NFAT-NanoLuc cells under mediation of H929 cells and K562 cells, respectively, by FACS**

| **Designation of antibody** | **Jurkat-NFAT-NanoLuc** | | | |
|---|---|---|---|---|
| | **H929** | | **K562** | |
| | **Maximum fluorescence intensity** | **EC50 (nM)** | **Maximum fluorescence intensity** | **EC50 (nM)** |
| Tri-17 | 205040 | 0.0004 | 74080 | The fit is poor |
| Tri-32 | 179680 | 0.05 | 44960 | The fit is poor |
| Tri-46 | 203800 | 0.09 | 53080 | The fit is poor |
| Tri-55 | 216920 | 0.07 | 36040 | The fit is poor |
| Tri-56 | 238080 | 0.02 | 96960 | The fit is poor |
| REGN5459 | 219840 | 0.75 | 17280 | The fit is poor |
| JNJ7564 | 194640 | 0.04 | 32920 | The fit is poor |
| antiFITC×CD3-Hab01 | 29720 | Negative | 35680 | Negative |
| Tri-52 | 212360 | 0.08 | 33040 | The fit is poor |
| Tri-59 | 214840 | 0.004 | 109800 | The fit is poor |
| Tri-60 | 178560 | 0.0004 | 84520 | The fit is poor |
| Tri-62 | 208280 | 0.001 | 99400 | The fit is poor |
| Tri-63 | 212240 | 0.08 | 7200 | The fit is poor |
| Tri-64 | 155920 | 0.16 | 12040 | The fit is poor |
| Tri-65 | 196200 | 0.22 | 7440 | The fit is poor |
| Tri-66 | 155560 | 0.29 | 10560 | The fit is poor |
| REGN5459 | 217520 | 0.64 | 22680 | The fit is poor |
| JNJ7564 | 188880 | 0.02 | 26760 | The fit is poor |
| antiFITC×CD3-Hab01 | 12280 | Negative | 18200 | Negative |
| Tri-29 | 163930 | 0.05 | 31520 | The fit is poor |
| Tri-35 | 143340 | 0.14 | 31330 | The fit is poor |
| Tri-38 | 206700 | 0.06 | 74840 | The fit is poor |
| Tri-41 | 175380 | 0.16 | 24880 | The fit is poor |
| REGN5459 | 181640 | 1.22 | 8040 | The fit is poor |
| JNJ7564 | 165410 | 0.09 | 37780 | The fit is poor |
| antiFITC×CD3-Hab01 | 11360 | Negative | 11840 | Negative |
| Tri-49 | 63610 | 0.74 | 34950 | The fit is poor |
| REGN5459 | 117990 | 2.08 | 9030 | The fit is poor |
| JNJ7564 | 90660 | 0.17 | 31340 | The fit is poor |
| antiFITC×CD3-Hab01 | 11470 | Negative | 19660 | Negative |

### Example 5 Detection of killing activity of T cells against tumor cells under mediation of anti-BCMA-GPRC5D-CD3 trispecific antibody in vitro and detection of cytokine production

In this experiment, three human multiple myeloma cell lines were selected as target cells for T cell killing experiment in vitro, among which NCI-H929 was a cell line with a high expression of both GPRC5D and BCMA, RPMI8226 was a cell line with a medium-low expression of GPRC5D and a medium expression of BCMA, and Molp-8 was a cell line with a high expression of GPRC5D and a low expression of BCMA. In order to facilitate the subsequent experimental detection, the above-mentioned three stable transformation cell lines stably expressing luciferase were constructed, hereinafter referred to as H929-Luc, RPMI8226-Luc and Molp-8-Luc (see 3.3 of example 3 for the construction method).

On the first day of the experiment, healthy donor PBMC (human peripheral blood mononuclear cells), which were cryopreserved in liquid nitrogen, were resuscitated, resuspended in a RPMI-1640 medium, and cultured in a culture flask overnight in a 5*%*CO₂, 37°C incubator for later use. On the second day, the H929-Luc, RPMI8226-Luc and Molp-8-Luc cells were taken to prepare a single cell suspension, respectively. Using a complete medium, the tumor cell density was adjusted to 1.33 × 10⁵ cells/ml, and the PBMC density was adjusted to 1.33 × 10⁶ cells/ml. 75 µl of the adjusted H929-Luc, RPMI8226-Luc or Molp-8-Luc was taken and uniformly mixed with 75 µl of the PBMC (the number of the tumor cells and PBMC in each well was 1 × 10⁴ cells and 1 × 10⁵ cells, respectively), and the cell mixing liquid was added to each well of a U bottom 96-well plate by a micropipette at 150 µl/well. The antibody was gradiently diluted with a complete medium (the initial concentration of the antibody was 100 nM, a dilution ratio of 7, and 8 concentration gradients), respectively, and the antibody diluents at different concentrations were added to each well at 100 µl/well, so that the final volume in each well was 250 µl. After uniformly mixing, the cells were placed in a 5%CO₂, 37°C incubator. After culture for 48 h, 100 µl of the supernatant was taken to detect cytokines IFNγ and TNFα in the cell medium supernatant. The remaining cells continued to be cultured in a 5*%*CO₂, 37°C incubator. After continuing the culture for 24 h, the cell viability was detected with Bright-Glo Luciferase kit (purchased from Promega, catalog number: E2620, refer to the product instructions for use). The well only added with PBMC was defined as a positive control with a killing rate of 100*%*, the cell in which the antibody concentration was 0 was defined as a blank negative control, the cell viability = (sample well reading value-positive well reading value)/(blank well reading value-positive well reading value) × 100*%*, and the results were calculated and plotted by GraphPad Prism 9.0 software.

The target cells used for the in vitro killing of some trispecific antibodies were Molp-8 cells that do not express luciferase, and the method for the mixed incubation of PBMC, tumor cells and antibodies was the same as above. After co-culture for 24 h, the cell culture plate was centrifuged at a rotary speed of 300 g, and 100 µl of the supernatant was transferred to detect the cytokines released from the cell medium supernatant. 100 µl of the remaining supernatant was transferred to a flat-bottomed 96-well plate, and the LDH value released by the cells in the remaining medium supernatant in each well was detected using a LDH detection kit (purchased from DO JINDO, catalog number: CK12, refer to the product instructions for use), and the killing percentage of the target cells was calculated according to the method in the product instructions of the kit.

There were two methods for detecting cytokines. One method was: detecting the cytokine release in a medium by a flow cytometer with Cytometric Bead Array human Th1/Th2/Th17 cytokine kit (purchased from BD, catalog number: 560484, refer to the product instructions of the kit for use); and the other method was: detecting cytokines IFNγ and TNFα in a cell medium supernatant by an ELISA method (the kit was purchased from BD, catalog number: 555142 and 55521, respectively, and refer to the product instructions of the kit for use).

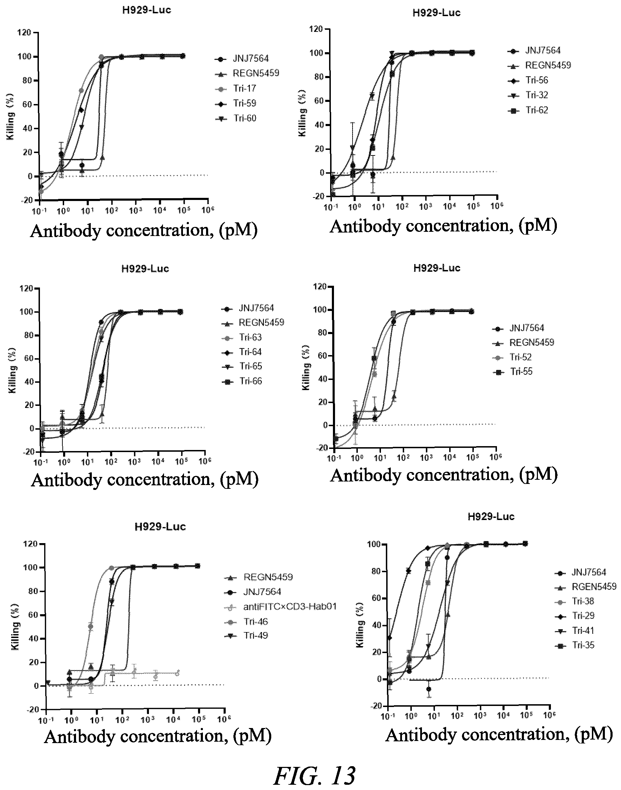

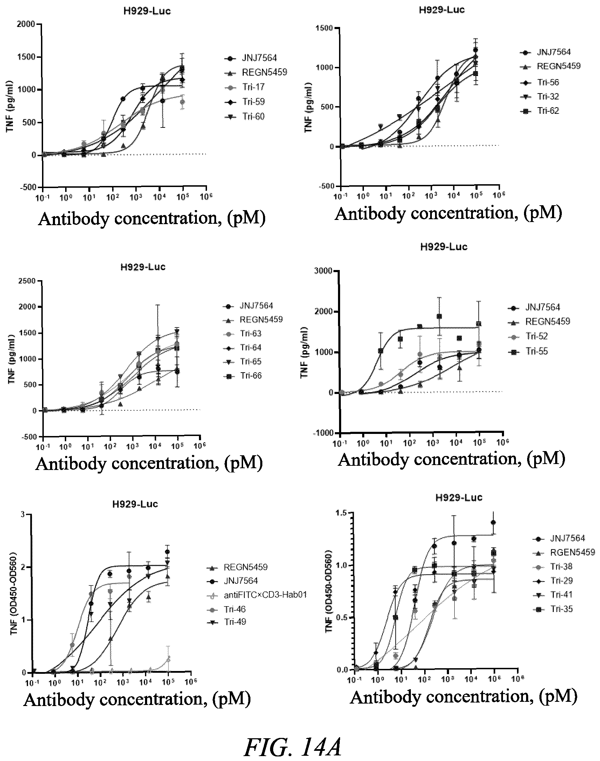

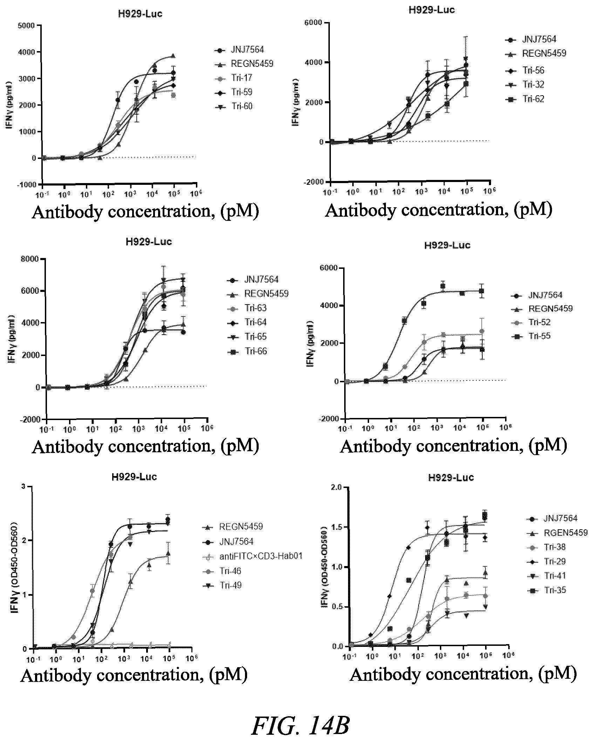

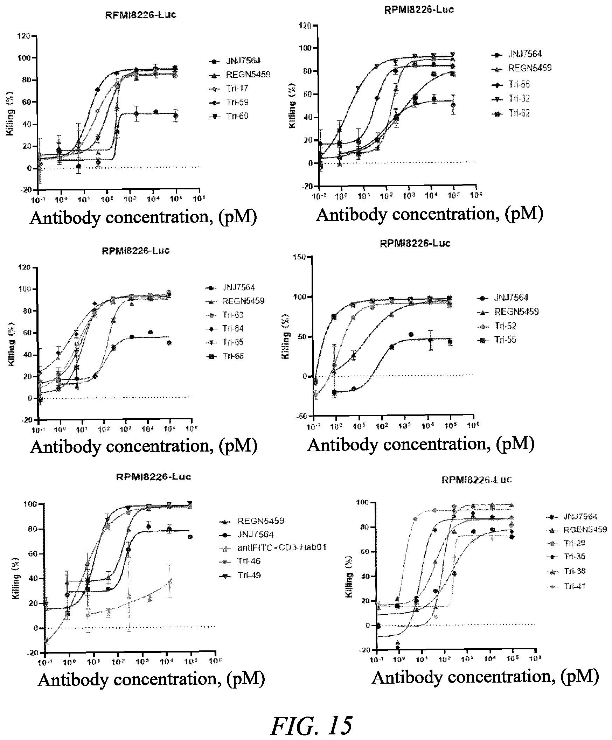

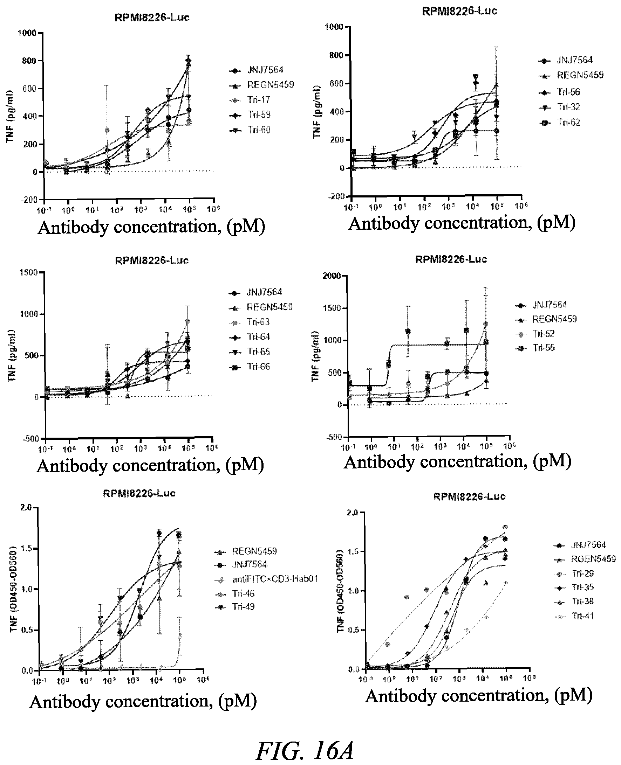

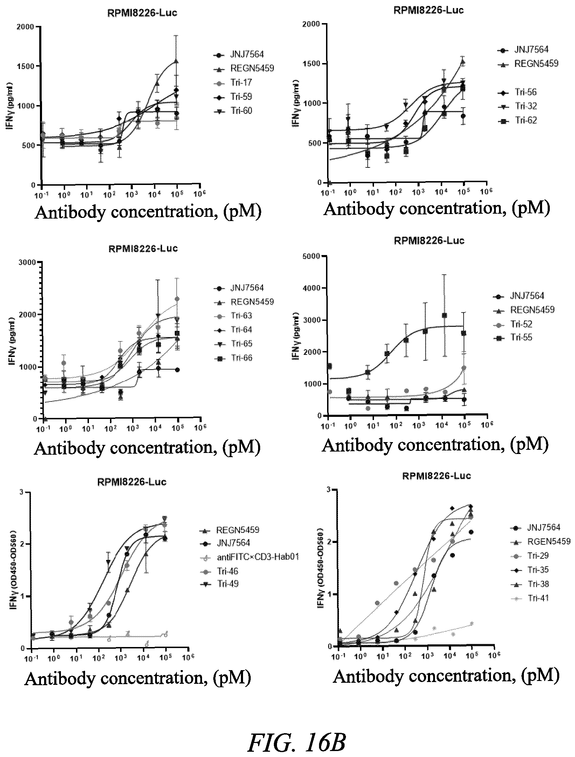

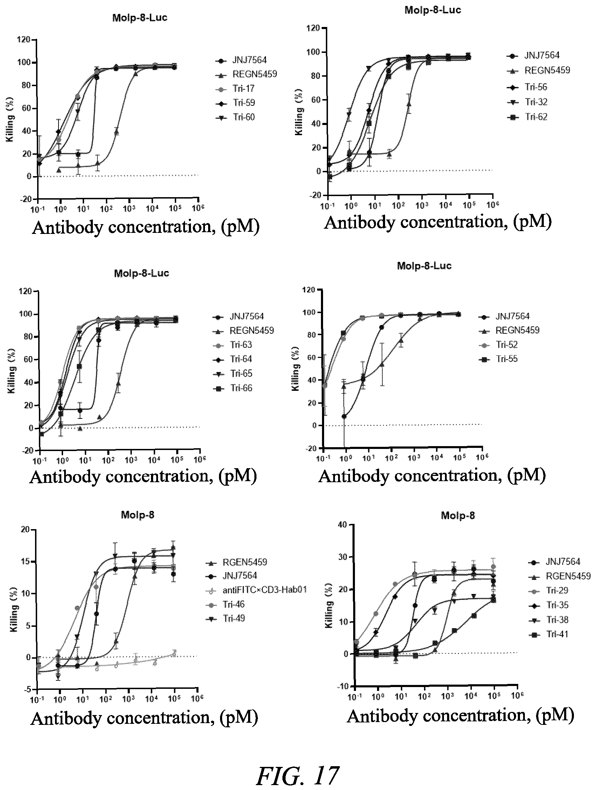

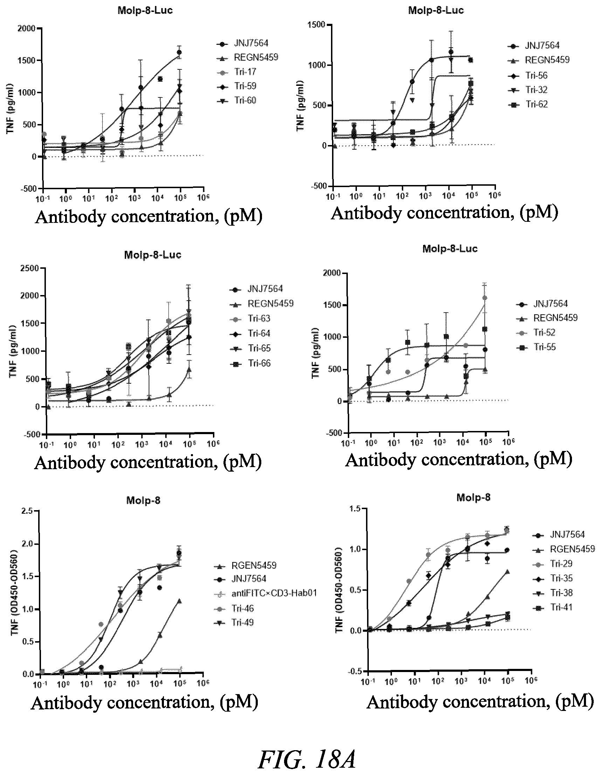

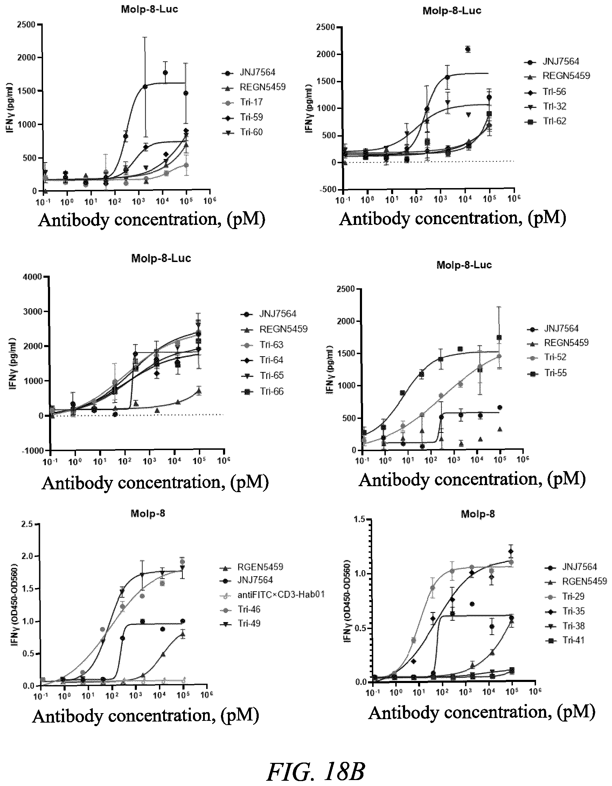

The killing results of T cells on tumor cells under the mediation of the trispecific antibodies were as shown in FIG. 13, FIG. 15, FIG. 17 and Table 12, respectively, indicating that the trispecific antibodies can produce a better or comparable killing effect on different tumor cells compared with the positive control, and the killing effects of T cells on Molp-8 under the mediation of the trispecific antibodies Tri-38 and Tri-41, respectively, were weaker than that of the positive control. For the cytokines TNFα and IFNγ secreted during the in vitro killing of tumor cells, the results were as shown in FIGs. 14A-14B, FIGs. 16A-16B and FIGs. 18A-18B, indicating that T cells can secrete a certain number of cytokines when killing tumor cells under the mediations of the trispecific antibodies Tri-29, Tri-35, Tri-46 and Tri-55.

**Table 12. Killing effects of T cells on H929-Luc, RPMI8226-Luc and Molp-8-Luc or Molp-8 cells, respectively, under mediation of trispecific antibodies**

| | H929-Luc | | RPMI8226-Luc | | Molp-8-Luc | |
|---|---|---|---|---|---|---|
| Designation of antibody | Maximum killing rate (*%*) | IC50 (nM) | Maximum killing rate (*%*) | IC50 (nM) | Maximum killing rate (*%*) | IC50 (nM) |
| JNJ7564 | 99.0 | 33.0 | 49.0 | 276.0 | 94.0 | 32.0 |
| REGN5459 | 99.0 | 58.0 | 84.0 | 275.0 | 95.0 | 419.0 |
| Tri-17 | 100.0 | 2.3 | 84.8 | 34.6 | 95.7 | 2.6 |
| Tri-59 | 101.0 | 3.4 | 88.8 | 17.4 | 97.2 | 1.5 |
| Tri-60 | 99.4 | 7.8 | 88.3 | 112.5 | 95.4 | 6.0 |
| JNJ7564 | 99.0 | 31.0 | 54.0 | 98.0 | 94.0 | 14.0 |
| REGN5459 | 100.0 | 64.0 | 89.0 | 187.0 | 94.0 | 280.0 |
| Tri-56 | 99.4 | 9.0 | 84.0 | 33.9 | 95.7 | 5.8 |
| Tri-32 | 100.9 | 2.3 | 92.1 | 2.0 | 95.4 | 0.9 |
| Tri-62 | 100.2 | 11.2 | 82.8 | 464.5 | 92.5 | 6.6 |
| JNJ7564 | 98.0 | 23.0 | 46.0 | 70.0 | 98.0 | 8.4 |
| REGN5459 | 98.0 | 73.0 | 94.0 | 20.0 | 100.0 | 136.0 |
| Tri-52 | 98.0 | 5.1 | 93.0 | 1.0 | 97.0 | 0.1 |
| Tri-55 | 98.0 | 3.9 | 96.0 | The fit is poor | 97.0 | 0.1 |
| JNJ7564 | 99.5 | 13.2 | 55.2 | 122.4 | 91.5 | 34.2 |
| REGN5459 | 99.6 | 78.7 | 90.0 | 170.7 | 95.5 | 383.0 |
| Tri-63 | 99.8 | 17.1 | 93.9 | 6.8 | 95.3 | 1.1 |
| Tri-64 | 99.8 | 52.1 | 93.7 | 3.6 | 95.5 | 1.5 |
| Tri-65 | 100.0 | 16.0 | 93.7 | 10.7 | 94.6 | 1.7 |
| Tri-66 | 100.3 | 46.0 | 92.0 | 11.6 | 93.3 | 3.6 |

| Designation of antibody | H929-Luc | | RPMI8226-Luc | | Molp-8 | |
|---|---|---|---|---|---|---|
| | Maximum killing rate (*%*) | IC50 (nM) | Maximum killing rate (*%*) | IC50 (nM) | Maximum killing rate (*%*) | IC50 (nM) |
| JNJ7564 | 100.0 | 26.0 | 77.6 | 260.2 | 14.0 | 40.0 |
| REGN5459 | 100.0 | 192.0 | 96.7 | 190.9 | 17.0 | 925.0 |
| antiFITC×C D3-Hab01 | 9.5 | The fit is poor | 24.2 | The fit is poor | 0.9 | The fit is poor |
| Tri-46 | 100.0 | 5.8 | 97.6 | 3.3 | 14.0 | 4.6 |
| Tri-49 | 100.0 | 29.0 | 98.0 | 12.4 | 16.0 | 11.0 |
| JNJ7564 | 99.0 | 32.0 | 77.0 | 214.0 | 24.0 | 35.0 |
| RGEN5459 | 100.0 | 52.0 | 97.0 | 92.0 | 23.0 | 974.0 |
| Tri-29 | 99.0 | 0.3 | 93.0 | 0.8 | 26.0 | 0.7 |
| Tri-35 | 99.0 | 2.1 | 86.0 | 9.2 | 24.0 | 2.3 |
| Tri-38 | 100.0 | 3.4 | 85.0 | 44.0 | 17.0 | 44.0 |
| Tri-41 | 101.0 | 20.0 | 72.0 | The fit is poor | 19.0 | 6155.0 |

## Claims

1. A multi-specific binding molecule, **characterized by** comprising:
(a) a first antigen binding molecule (ABM1) that specifically binds to a first tumor-associated antigen (TAA1);
(b) a second antigen binding molecule (ABM2) that specifically binds to a second tumor-associated antigen (TAA2); and
(c) a third antigen binding molecule (ABM3) that specifically binds to an antigen expressed on a human immune cell, optionally the TAA1 and/or the TAA2 being expressed on a cancerous B cell.

2. The multi-specific binding molecule according to claim 1, **characterized in that** the cancerous B cell is a plasma cell-derived or B cell-derived, and optionally the immune cell is selected from a T cell, a NK cell or a macrophage.

3. The multi-specific binding molecule according to claim 1 or 2, **characterized in that** the TAA1 and TAA2 are each independently selected from BCMA and GPRC5D, and/or the antigen expressed on the human immune cell is CD3.

4. The multi-specific binding molecule according to any one of claims 1 to 3, **characterized in that** the ABM1, ABM2 and ABM3 are each independently selected from an antibody, an antibody fragment, F(ab')₂, Fab', Fab, Fv, scFv, a nanobody or VHH.

5. The multi-specific binding molecule according to any one of claims 1 to 4, **characterized in that**, the multi-specific binding molecule comprises four polypeptide chains: a first heavy chain, a first light chain, a second heavy chain and a second light chain.

6. The multi-specific binding molecule according to claim 5, **characterized in that** (1) the first heavy chain and the second heavy chain are the same and have the structure as shown below:
(a) VHₐ₋CH1-L1-VHH_{b}-Fc-L2-VH_{CD3}-L3-VL_{CD3};
(b) VHₐ-CH1-L1-VH_{CD3}-L2-VL_{CD3}-Fc-L3-VHH_{b};
(c) VHH_{b}-L1-VHₐ-CH1-L2-VH_{CD3}-L3-VL_{CD3}-Fc;
(d) VHₐ-CH1-L1-VHH_{b}-L2-VH_{CD3}-L3-VL_{CD3}-Fc; or
(e) VHₐ-CH1-L1-VH_{b}-L2-VL_{b}-Fc-L3-VH_{CD3}-L4-VL_{CD3};
and/or, (2) the first light chain and the second light chain are the same and have the structure as shown below: VLₐ-CL;
wherein VHₐ and VLₐ are a heavy chain variable region and a light chain variable region that specifically bind to GPRC5D, respectively, VHH_{b} is a nanobody that specifically binds to BCMA, VH_{b} and VL_{b} are a heavy chain variable region and a light chain variable region that specifically bind to BCMA, respectively, VH_{CD3} and VL_{CD3} are a heavy chain variable region and a light chain variable region that specifically bind to CD3, respectively, Fc is a Fc region of any antibody, and L1, L2, L3 and L4 are the same or different linkers, respectively.

7. The multi-specific molecule according to claim 5, **characterized in that** (1) the first heavy chain and the second heavy chain are different and have the structure as shown below:
(a) a first heavy chain VH_{A}-CH1-L1-VHH_{b}-Fc1 and a second heavy chain VHₐ-CH1-L2-VH_{CD3}-L3-VL_{CD3}-Fc2; ;
(b) a first heavy chain VHH_{b}-L1-VHₐ-CH1-Fc1 and a second heavy chain VHₐ-CH1-L2-VH_{CD3}-L3-VL_{CD3}-Fc2;
(c) a first heavy chain VHₐ₋CH1-L1-VH_{b}-L2-VL_{b}-Fc1 and a second heavy chain VHₐ-CH1-L3-VH_{b}-L4-VL_{b}-Fc2-L5-VH_{CD3}-L6-VL_{CD3};
(d) a first heavy chain VHₐ-CH1-L1-VHH_{b}-Fc1 and a second heavy chain VHₐ-CH1-L2-VHH_{b}-Fc2-L3-VH_{CD3}-L4-VL_{CD3}; or
(e) a first heavy chain VHH_{b}-L1-VHₐ-CH1-Fc1 and a second heavy chain VHH_{b}-L2-VHₐ-CH1-Fc2-L3-VH_{CD3}-L4-VL_{CD3};
optionally, the first heavy chain further has a Flag-tag at the carboxyl terminus and/or the second heavy chain further has a His-tag at the carboxyl terminus in the above-mentioned (a)-(e);
and, (2) the first light chain and the second light chain are the same and have the structure as shown below: VLₐ-CL;
wherein VHₐ and VLₐ are a heavy chain variable region and a light chain variable region that specifically bind to GPRC5D, respectively, VHH_{b} is a nanobody that specifically binds to BCMA, VH_{b} and VL_{b} are a heavy chain variable region and a light chain variable region that specifically bind to BCMA, respectively, VH_{CD3} and VL_{CD3} are a heavy chain variable region and a light chain variable region that specifically bind to CD3, respectively, and L1, L2, L3, L4, L5 and L6 are the same or different linkers, respectively.

8. The multi-specific binding molecule according to any one of claims 1 to 4, **characterized in that** the multi-specific binding molecule comprises three polypeptide chains: a first heavy chain, a second heavy chain and a light chain, wherein (1) the first heavy chain and the second heavy chain have the structure as shown below:
(a) a first heavy chain VHH_{b}-L1-VHH_{b}-Fc1 and a second heavy chain VHₐ-CH1-L2-VH_{CD3}-L3-VL_{CD3}-Fc2;
(b) a first heavy chain VHH_{b}-L1-VH_{CD3}-L2-VL_{CD3}-Fc1 and a second heavy chain VHₐ-CH1-Fc2; or
(c) a first heavy chain VHH_{b}-Fc1 and a second heavy chain VHₐ-CH1-L1-VH_{CD3}-L2-VL_{CD3}-Fc2;
optionally, the first heavy chain further has a Flag-tag at the carboxyl terminus and/or the second heavy chain further has a His-tag at the carboxyl terminus in the above-mentioned (a)-(c);
and, (2) the light chain has the structure as shown below: VLₐ-CL;
wherein VHₐ and VLₐ are a heavy chain variable region and a light chain variable region that specifically bind to GPRC5D respectively, VHH_{b} is a nanobody that specifically binds to BCMA, VH_{b} and VL_{b} are a heavy chain variable region and a light chain variable region that specifically bind to BCMA respectively, VH_{CD3} and VL_{CD3} are a heavy chain variable region and a light chain variable region that specifically bind to CD3 respectively, Fc1 and Fc2 are each a Fc region of any antibody, and L1, L2 and L3 are the same or different linkers, respectively.

9. The multi-specific binding molecule according to any one of claims 6 to 8, **characterized in that** Fc1 and Fc2 of the multi-specific binding molecule are different, Fc1 is knob-Fc and Fc2 is hole-Fc; or, Fc1 is hole-Fc, and Fc2 is knob-Fc; preferably, wherein the knob-Fc comprises a T366W mutation, and/or the hole-Fc comprises a T366S, L368A and/or Y407V mutation; and preferably, Fc1 and Fc2 are Fc regions of IgG1 or IgG4.

10. The multi-specific binding molecule according to any one of claims 6 to 8, **characterized in that** Fc1 and Fc2 of the multi-specific molecule are the same or different, and have an amino acid mutation that changes effector functions; preferably, the mutation that changes effector functions comprises an S228P, F234A, L234A and/or L235A mutation; and more preferably, the mutation that changes effector functions comprises: (a) a L234A and/or L235A mutation of IgG1 Fc; and/or (b) an S228P, F234A and/or L235A mutation of IgG4 Fc.

11. The multi-specific binding molecule according to any one of claims 1 to 3, **characterized in that** ABM3 is an anti-CD3 antibody or an antigen binding fragment thereof, and comprises a light chain variable region and a heavy chain variable region: (a) the light chain variable region comprises LCDR1, LCDR2 and LCDR3, and the LCDR1, LCDR2 and LCDR3 have amino acid sequences as shown in SEQ ID NOs: 54-56, or having 1, 2, 3 or more amino acid insertions, deletions and/or substitutions compared to SEQ ID NOs: 54-56; and (b) the heavy chain variable region comprises HCDR1, HCDR2 and HCDR3, and the HCDR1, HCDR2 and HCDR3 have amino acid sequences as shown in SEQ ID NOs: 51-53, or having 1, 2, 3 or more amino acid insertions, deletions and/or substitutions compared to SEQ ID NOs: 51-53.

12. The multi-specific binding molecule according to claim 11, **characterized in that** the light chain variable region comprises an amino acid sequence as shown in SEQ ID NO: 10 or comprises an amino acid sequence having a 90*%*, 95*%*, 96*%*, 97*%*, 98*%*, 99*%* or more identity to SEQ ID NO: 10, and/or the heavy chain variable region comprises an amino acid sequence as shown in SEQ ID NO: 9 or comprises an amino acid sequence having a 90*%*, 95*%*, 96*%*, 97*%*, 98*%*, 99*%* or more identity to SEQ ID NO: 9.

13. The multi-specific binding molecule according to claim 12, **characterized in that** the ABM3 is scFv.

14. The multi-specific binding molecule according to any one of claims 1 to 3, **characterized in that** the TAA1 and the TAA2 are selected from the following group: (a) TAA1 is BCMA, and TAA2 is GPRC5D; or (b) TAA1 is GPRC5D, and TAA2 is BCMA.

15. The multi-specific binding molecule according to claim 14, **characterized in that** the antigen binding molecule that specifically binds to GPRC5D in the multi-specific binding molecule comprises a light chain variable region and a heavy chain variable region of an antibody: (a) the light chain variable region comprises LCDR1, LCDR2 and LCDR3, the LCDR1, LCDR2 and LCDR3 have amino acid sequences as shown in SEQ ID NOs: 42-44, or having 1, 2, 3 or more amino acid insertions, deletions and/or substitutions compared to SEQ ID NOs: 42-44, the heavy chain variable region comprises HCDR1, HCDR2 and HCDR3, and the HCDR1, HCDR2 and HCDR3 have amino acid sequences as shown in SEQ ID NOs: 39-41, or having 1, 2, 3 or more amino acid insertions, deletions and/or substitutions compared to SEQ ID NOs: 39-41; or (b) the light chain variable region comprises LCDR1, LCDR2 and LCDR3, the LCDR1, LCDR2 and LCDR3 have amino acid sequences as shown in SEQ ID NOs: 48-50, or having 1, 2, 3 or more amino acid insertions, deletions and/or substitutions compared to SEQ ID NOs: 48-50, the heavy chain variable region comprises HCDR1, HCDR2 and HCDR3, and the HCDR1, HCDR2 and HCDR3 have amino acid sequences as shown in SEQ ID NOs: 45-47, or having 1, 2, 3 or more amino acid insertions, deletions and/or substitutions compared to SEQ ID NOs: 45-47.

16. The multi-specific binding molecule according to claim 15, **characterized in that** :
(a) the light chain variable region comprises an amino acid sequence as shown in SEQ ID NO: 6 or comprises an amino acid sequence having a 90*%*, 95*%*, 96*%*, 97*%*, 98*%*, 99*%* or more identity to SEQ ID NO: 6, and/or the heavy chain variable region comprises an amino acid sequence as shown in SEQ ID NO: 5 or comprises an amino acid sequence having a 90*%*, 95*%*, 96*%*, 97*%*, 98*%*, 99*%* or more identity to SEQ ID NO: 5; or
(b) the light chain variable region comprises an amino acid sequence as shown in SEQ ID NO: 8 or comprises an amino acid sequence having a 90*%*, 95*%*, 96*%*, 97*%*, 98*%*, 99*%* or more identity to SEQ ID NO: 8, and/or the heavy chain variable region comprises an amino acid sequence as shown in SEQ ID NO: 7 or comprises an amino acid sequence having a 90*%*, 95*%*, 96*%*, 97*%*, 98*%*, 99*%* or more identity to SEQ ID NO: 7.

17. The multi-specific binding molecule according to claim 16, **characterized in that** the antigen binding molecule that specifically binds to GPRC5D in the multi-specific binding molecule is Fab.

18. The multi-specific binding molecule according to claim 14, **characterized in that** the antigen binding molecule that specifically binds to BCMA in the multi-specific binding molecule comprises a heavy chain variable region of an antibody, the heavy chain variable region comprises HCDR1, HCDR2 and HCDR3, and the HCDR1, HCDR2 and HCDR3 have amino acid sequences as shown in SEQ ID NOs: 27-29 or as shown in SEQ ID NOs: 30-32, or having 1, 2, 3 or more amino acid insertions, deletions and/or substitutions compared to SEQ ID NOs: 27-29 or SEQ ID NOs: 30-32.

19. The multi-specific binding molecule according to claim 18, **characterized in that** the heavy chain variable region comprises an amino acid sequence as shown in SEQ ID NO: 1 or SEQ ID NO: 2, or comprises an amino acid sequence having a 90*%*, 95*%*, 96*%*, 97*%*, 98*%*, 99*%* or more identity to SEQ ID NO: 1 or SEQ ID NO: 2.

20. The multi-specific binding molecule according to claim 19, **characterized in that** the antigen binding molecule that specifically binds to BCMA is VHH.

21. The multi-specific binding molecule according to claim 14, **characterized in that** the antigen binding molecule that specifically binds to BCMA in the multi-specific binding molecule comprises a light chain variable region and a heavy chain variable region of an antibody: (a) the light chain variable region comprises LCDR1, LCDR2 and LCDR3, and the LCDR1, LCDR2 and LCDR3 have amino acid sequences as shown in SEQ ID NOs: 36-38, or having 1, 2, 3 or more amino acid insertions, deletions and/or substitutions compared to SEQ ID NOs: 36-38; and (b) the heavy chain variable region comprises HCDR1, HCDR2 and HCDR3, and the HCDR1, HCDR2 and HCDR3 have amino acid sequences as shown in SEQ ID NOs: 33-35, or having 1, 2, 3 or more amino acid insertions, deletions and/or substitutions compared to SEQ ID NOs: 33-35.

22. The multi-specific binding molecule according to claim 21, **characterized in that** the light chain variable region comprises an amino acid sequence as shown in SEQ ID NO: 4 or comprises an amino acid sequence having a 90*%*, 95*%*, 96*%*, 97*%*, 98*%*, 99*%* or more identity to SEQ ID NO: 4, and/or the heavy chain variable region comprises an amino acid sequence as shown in SEQ ID NO: 3 or comprises an amino acid sequence having a 90*%*, 95*%*, 96*%*, 97*%*, 98*%*, 99*%* or more identity to SEQ ID NO: 3.

23. The multi-specific binding molecule according to claim 22, **characterized in that** the antigen binding molecule that specifically binds to BCMA is scFv.

24. The multi-specific binding molecule according to any one of claims 1 to 23, **characterized in that** the ABM1, ABM2 and/or ABM3 is chimeric, humanized or fully humanized.

25. The multi-specific binding molecule according to claim 24, **characterized by** further comprising an Fc fragment of human IgG1, IgG2, IgG3 or IgG4; preferably comprising an Fc fragment of human IgG1 or human IgG4.

26. The multi-specific binding molecule according to claim 25, **characterized in that** the Fc fragment comprises the same or different Fc1 and Fc2.

27. The multi-specific binding molecule according to claim 25, **characterized in that** the Fc fragment has an amino acid mutation that changes effector functions; preferably, the amino acid mutation that changes effector functions comprises an S228P, F234A, L234A and/or L235A mutation; and more preferably, the amino acid mutation that changes effector functions comprises: (a) a L234A and/or L235A mutation of IgG1 Fc; and/or (b) an S228P, F234A and/or L235A mutation of IgG4 Fc.

28. The multi-specific binding molecule according to claim 27, **characterized in that** the different Fc1 and Fc2 have amino acid mutations that promote heterodimerization; preferably, the amino acid mutations that promote heterodimerization comprise a Knob-into-hole (KIH) mutation; and more preferably, the Knob-into-hole mutation comprises: (a) a T366W mutation in any Fc region; and (b) a T366S, L368A and/or Y407V mutation in another Fc region.

29. The multi-specific binding molecule according to claim 28, **characterized in that** the Fc1 further comprises a His-tag, and the Fc2 further comprises a Flag-tag; or the Fc1 further comprises a Flag-tag, and the Fc2 further comprises a His-tag.

30. The multi-specific binding molecule according to any one of claims 11 to 29, **characterized in that** the multi-specific binding molecule is trivalent, tetravalent, pentavalent or hexavalent.

31. An isolated nucleic acid, **characterized by** encoding the multi-specific binding molecule according to any one of claims 1 to 30.

32. A recombinant vector, **characterized by** comprising the isolated nucleic acid according to claim 31.

33. A host cell, **characterized by** comprising the isolated nucleic acid according to claim 31 or the recombinant vector according to claim 32, wherein preferably, the host cell is a eukaryotic cell or a prokaryotic cell; more preferably, the host cell is derived from a mammalian cell, a yeast cell, an insect cell, Escherichia coli and/or Bacillus subtilis; and more preferably, the host cell is Expi293 cell.

34. A method for preparing a multi-specific binding molecule, **characterized by** comprising: culturing the host cell according to claim 33 under appropriate conditions, and isolating the multi-specific binding molecule.

35. A pharmaceutical composition, **characterized in that** the pharmaceutical composition comprises the multi-specific binding molecule according to any one of claims 1 to 30, the isolated nucleic acid according to claim 31, the recombinant vector according to claim 32, the host cell according to claim 33, or a product prepared by the method according to claim 34, and one or more pharmaceutically acceptable carriers, diluents, buffers or excipients; preferably, the pharmaceutical composition further comprises an additional cancer therapeutic agent, preferably, the pharmaceutical composition is used for treating and/or preventing cancers, and preferably the cancer is B-cell lymphoma; and more preferably, the B-cell lymphoma is multiple myeloma.

36. A method for activating T cells, **characterized by** comprising: administering to a subject a therapeutically effective amount of the multi-specific binding molecule according to any one of claims 1 to 30 or the pharmaceutical composition according to claim 35.

37. The multi-specific binding molecule according to any one of claims 1 to 30, the isolated nucleic acid according to claim 31, the host cell according to claim 33, a product prepared by the method according to claim 34 or the pharmaceutical composition according to claim 35, for use in the preparation of a drug for preventing and/or treating cancers, or for use in the treatment and/or prevention of cancers;
preferably, the cancer is B-cell lymphoma; and more preferably, the B-cell lymphoma is multiple myeloma.

38. A method for treating and/or preventing cancers, **characterized by** comprising: administering to a subject a therapeutically effective amount of the multi-specific binding molecule according to any one of claims 1 to 30, the isolated nucleic acid according to claim 31, the host cell according to claim 33, a product prepared by the method according to claim 34 or the pharmaceutical composition according to claim 35,
wherein preferably, the cancer is B-cell lymphoma; and more preferably, the B-cell lymphoma is multiple myeloma.
